# EUROPEAN PATENT SPECIFICATION

(11) **EP 2 007 884 B1**
(45) Date of publication and mention of the grant of the patent: **16.05.2012**
(21) Application number: 07730683.5
(22) Date of filing: 12.04.2007
(51) Int. Cl.: C12N 9/42, C12N 15/62, C12N 15/56, C12N 15/63, D06M 16/00, C11D 3/386, A23K 1/165

(54) **CELLULASE FUSION PROTEINS AND THEIR USE**
CELLULASE FUSIONSPROTEINE UND DEREN VERWENDUNGEN
PROTEINES DE FUSION DE CELLULASES ET LEUR UTILISATION

(30) Priority: 13.04.2006 FI 20065234; 13.04.2006 US 404065
(43) Date of publication of application: 31.12.2008
(73) Proprietor: AB Enzymes Oy, 05200 Rajamäki (FI)
(72) Inventor: ALAPURANEN, Marika, FI-05200 Rajamäki (FI); VALTAKARI, Leena, FI-05200 Rajamäki (FI); KALLIO, Jarno, FI-04400 Järvenpää (FI); OJAPALO, Pentti, FI-04360 Tuusula (FI); VEHMAANPERÄ, Jari, FI-01800 Klaukkala (FI)
(74) Representative: Boije-Backman, Solveig Magdalena
(86) International application number: PCT/FI2007/050196
(87) International publication number: WO 2007/118935

(56) References cited:
- WO-A1-96/29397
- WO-A1-99/57260
- WO-A1-2007/071820
- WO-A2-03/000941
- ITO J. ET AL.: 'Improvement of cellulose-degrading ability of a yeast strain displaying Trichoderma reesei endoglucanase II by recombination of cellulose-binding domains' BIOTECHNOL. PROG. vol. 20, 2004, pages 688 - 691, XP002405964
- DATABASE UNIPROT [Online] LIU S.A. ET AL.: 'Molecular cloning and sequence analysis of the cellulose gene (cbh1) encoding cellobiohydrolase, from the moderately thermophilic fungus Chaetomium thermophilum CT2', XP008138583 Retrieved from EBI Database accession no. (Q5G2D5)
- DATABASE UNIPROT [Online] DABROWSKI S. ET AL.: 'Cloning, expression and purification of CBHI exoglucanase from Chaetomium thermophilum DSM1495', XP008138584 Retrieved from EBI Database accession no. (Q69212)
- LI Y.-L. ET AL.: 'Purification and characterization of a cellobiohydrolase from the thermophilic fungus Chaetomium thermophilum CT2' WEISHENGWU XUEBAO vol. 46, no. 1, 04 February 2006, pages 143 - 146, XP009134289
- LO LEGGIO L. ET AL.: 'The 1.62 A structure of Thermoascus aurantiacus endoglucanase: completing the structural picture of subfamilies in glycoside hydrolase family 5' FEBS LETTER vol. 523, 2002, pages 103 - 108, XP027233208

## Description

### Field of the Invention

The present invention relates to enzyme technology, and more precisely to cellulase fusion proteins comprising a catalytic domain and a cellulose binding domain. The fusion proteins may be produced by recombinant techniques using appropriate polynucleotides, expressing vectors and host cells, which are also encompassed by the invention. The fusion proteins and enzyme preparations thereof are useful in treating cellulosic material, such as textile material. In addition the fusion proteins may be used in pulp and paper industry, oil extraction from plants, detergent compositions, or for improving the quality of animal feed. The invention thus further relates to a process for treating cellulosic material with the fusion protein, and especially to processes for biostoning or biofinishing fabrics or garments, especially denim. The invention still further relates to detergent compositions and animal feed containing the fusion proteins.

### Background of the Invention

Cellulose is the major structural component of higher plants and occurs naturally in almost pure form only in cotton fiber. It provides plant cells with high tensile strength helping them to resist mechanical stress and osmotic pressure. Cellulose is a linear polysaccharide of glucose residues connected by ß-1,4-linkages. In nature cellulose is usually associated with lignin and hemicelluloses. Cellulosic material is degraded in nature by a number of various organisms including bacteria and fungi. The biological conversion of cellulose to glucose generally requires three major groups of enzymes: cellobiohydrolases (CBH), endoglucanases (EG) and beta-glucosidases (BG).

There is a wide spectrum of industrial applications of cellulases. In the textile industry, cellulases are used in denim finishing to create a fashionable stone washed appearance in denim cloths in a biostoning process. Cellulases are also used, for instance, to clean fuzz and prevent formation of pills on the surface of cotton garments. In detergent industry cellulases are used to brighten colors and to prevent graying and pilling of garments. Cellulases are further used in food industry and animal feed manufacturing, and they have a great potential in the pulp and paper industry, for instance, in deinking to release ink from fiber surfaces and in improving pulp drainage.

Industrially used cellulases are often mixtures of enzymes having a variety of activities and substrate specificities. The commercial enzyme preparations often comprise all three cellulase activities i.e. CBH, EG and BG. In addition the unique properties of each cellulase make some more suitable for certain purposes than others, and therefore efforts have also been made to obtain and use cellulases having only the desired activities. The most widely used cellulases of fungal origin are derived from *Trichoderma reesei.* However, also other fungal sources have been suggested e.g. in US 5,457,046.

Cellulases applied in denim treatment are usually divided into two main groups: acid and neutral cellulases. Acid cellulases typically operate at pH 4.0 to 5.5 and neutral cellulases in the range of pH 6 to 8. Cellulases having characteristics of both the acid and neutral group can be called hybrid cellulases. Acid cellulases used in biostoning mainly originate from *Trichoderma reesei* (sexual form *Hypocrea jecorina*) and the neutral cellulases come from a variety of fungi, including genera of *Melanocarpus, Humicola, Myceliophthora, Fusarium, Acremonium,* and *Chrysosporium* (Haakana *et al.* 2004). *T. reesei* enzymes include, e.g., cellulases from the glycosyl hydrolase family 5 (endoglucanase II, EGII), family 7 (cellobiohydrolase I, CBHI) and family 12 (endoglucanase III, EGIII; Ward *et al.* 1993), and the neutral cellulases, most often endoglucanases, from family 45 and family 7 (Henrissat, 1991; Henrissat and Bairoch, 1993, 1996).

US 5,874,293 discloses an improved cellulase composition comprising elevated amounts of EGII endoglucanase of *T. reesei* for treating cellulose-containing textiles. The composition improved color properties, increased lightness and visual appearance and reduced pilling tendencies. WO97/14804 discloses one 20 kDa and one 50 kDa cellulase with endoglucanase activity derived from *Melanocarpus* sp. that are especially useful in the textile and detergent industry. Fusion proteins containing the 20K- or 50K-proteins linked to a cellulose binding domain preferably from *Trichoderma reesei* are suggested for creating new enzyme properties. No specific examples are given, nor are the desired properties described.

However, there is still a need for improved cellulases, including endoglucanases that are more efficient in fabric treatment and in other fields, where cellulases traditionally are used. In particular, there is a continuous need for more efficient cellulases to improve the process economics. The present invention aims to meet these needs.

In textile industry a "stone washed" look or an abraded look has been denim producers' interest in recent years. Traditional stone washing with pumice stones reduces the strength of fabric and burdens the laundering apparatuses. There is a trend towards enzymatic denim finishing processes, and cellulases have replaced or are being used together with pumice stones to give the fabric its desired "worn" look. Controlled enzyme treatments result in less damage to the garments and machines and eliminate the need for disposal of stones.

A general problem associated with enzymatic stone washing is backstaining caused by redeposition of removed Indigo dye during or after abrasion. The redepositioning of Indigo dye reduces the desired contrast between the white and indigo dyed yarns and it can be most easily noted on the reverse side of denim and the interior pockets (as increased blueness). On the face side this may be seen as reduced contrast between dyed areas and areas from which dye has been removed during biostoning. Backstaining can be reduced by using antibackstaining agents such as nonionic ethoxylated alcohols during the treatment or adding bleaching agents during rinsing steps. The nature of the enzyme has an impact on backstaining. Generally neutral cellulases backstain less than acid cellulases.

WO97/09410 describes that the addition of a certain type of cellulase to another cellulase having abrading activity reduces backstaining. The additional cellulase belongs to family 5 or 7, but it has no significant abrading effect in itself. Preferably said additional cellulase originates from *Bacillus* or *Clostridium.*

US 5,916,799 discloses cellulase compositions containing both cellobiohydrolases and endoglucanases that have been subjected to limited proteolysis to separate the core and binding domains of the enzymes. The obtained enzyme compositions were found to reduce backstaining. WO94/07983 relates to the finding that redeposition of colorant onto fabric during the stone washing process can be reduced by employing a fungal cellulase composition, which is substantially free of cellobiohydrolase type components. WO96/23928 discloses treatment of cellulose containing fabrics with truncated cellulase enzymes. The truncated enzymes lacking cellulose binding domain (CBD) were found to reduce redeposition of dye and increase abrasion.

The general conclusion of the three references cited above could be that the cellulose binding domain has a negative effect on backstaining. However, the present invention now provides a cellulase construction with low backstaining property, despite the presence of a cellulose binding domain.

### Summary of the Invention

The present invention is based on the surprising finding that the effect of endoglucanses could be significantly enhanced by coupling thereto a particular cellulose binding domain without impairing the backstaining properties.

One object of the present invention is to provide novel endoglucanase fusion proteins having improved hydrolytic properties for use in textile industry, especially in stone washing denim. The fusion proteins of the invention have the advantage of being active at both acid and neutral pH values, and they have highly improved performance in biostoning applications. When used in treating denim, the fusion proteins provide a low backstaining effect. With the improved efficiency of the endoglucanases of the invention, the use of the enzymes is significantly more economical. Additional advantages are achieved also in terms of logistics and the storage of the enzyme products, because smaller amounts of the enzyme product are needed. The fusion proteins are also useful in detergent compositions, as well as in other fields such as feed industry, oil extraction from plants, or pulp and paper industry.

A further object of the present invention is to provide polynucleotides encoding the novel endoglucanase fusion proteins.

A still futher object of the present invention is to provide a method for producing the fusion proteins.

A still further object of the present invention is to provide novel expression vectors containing such polynucleotides, useful for the production of the endoglucanase fusion proteins, as well as novel hosts transformed with said expression vectors.

A still further object of the present invention is to provide enzyme preparations, which contain one or more novel endoglucanase fusion proteins.

A still further object of the present invention is to provide methods of treating cellulosic material with the fusion protein e.g for use in textile, detergent, animal feed, oil extraction from plants, or pulp and paper industry, and particularly for finishing of textiles, especially for biostoning and biofinishing of denim.

A still further object of the present invention is to provide animal feed or detergent compositions containing the fusion proteins.

The present invention relates to a cellulase fusion protein comprising a first amino acid sequence of an endoglucanase core having at least 75% identity to SEQ ID NO:2, or a fragment thereof having cellulase activity, and a second amino acid sequence comprising a linker and cellulose binding domain (CBD) having at least 75% identity to SEQ ID NO: 15, or a fragment thereof having cellulose binding activity.

The invention is further directed to an isolated polynucleotide selected from the group consisting of:
a) a nucleotide sequence of SEQ ID NO: 3, or a sequence encoding a cellulase fusion protein of claim 1,
b) a complementary strand of a).

The invention still further relates to an expression vector comprising said nucleotide sequence, and to a host cell comprising the expression vector, as well as to an enzyme preparation comprising the fusion protein.

The invention also encompasses a method of producing the fusion protein, comprising the steps of transforming a host cell with an expression vector encoding said fusion protein and culturing said host cell under conditions enabling expression of said fusion protein, and optionally recovering and purifying the fusion protein produced.

The invention still further encompasses a process for treating cellulosic material, wherein said process comprises contacting the cellulosic material with the fusion protein.

The invention is also directed to a process for biostoning, which process comprises the step of contacting the cellulase fusion protein or the enzyme preparation with denim fabric or garments, and to a process for biofinishing, which comprises the step of contacting the cellulase fusion protein or the enzyme preparation with textile materials like fabrics or garments or yam.

Eventually the invention is directed to a detergent composition comprising the fusion protein and detergent auxiliaries, to animal feed comprising the fusion protein, and to an *Eschrichia coli* strain having accession number E. coli DSM 18159.

Specific embodiments of the invention are set forth in the dependent claims.

Other objects, details and advantages of the present invention will become apparent from the following drawings, detailed description and examples.

### Brief Description of the Drawings

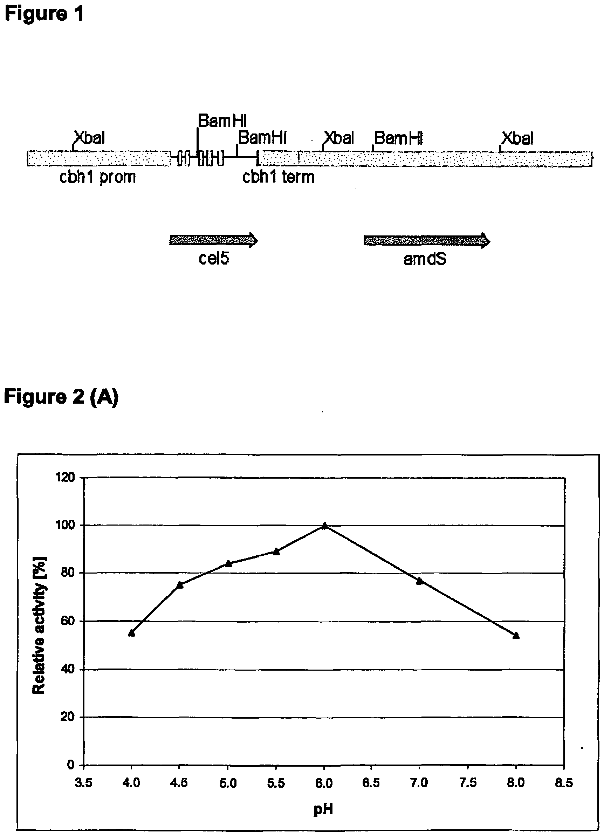

Figure 1 illustrates a schematic picture of the expression cassette used in transformation of *T. reesei* protoplasts for production of *T. aurantiacus* EG28 or EG28+CtCBD cellulase. The *cel*5A or *cel*5A_Ct *cel*7AlinkerCBD gene is under control of *T*. *reesei cbh*l promoter (cbhl prom) and transcription termination is ensured with the addition of the *cbh*l terminator (cbhl term). The *amd*S gene (amdS) is included for selection of transformants. The expression cassette for EG28 and EG28+CtCBD production was isolated as a 8.6 kb *Not*I fragment from pALK1930 or as a 8.9 kb *Not*I fragment from pALK1948, respectively.
Figure 2 illustrates the pH dependency of the heterologously produced *T. aurantiacus* EG28 cellulase by determining from the culture supernatant using CMC as substrate in a 10 min reaction at 50°C (A). Temperature optimum of EG28 cellulase was determined at optimal pH (6.0). The reaction containing CMC as substrate was performed for 10 min (B). The pH and temperature optimum of the EG28+CtCBD fusion protein was determined to be the same as those of the wild-type EG28 cellulase.
Figure 3 shows the biostoning effect of EG28 cellulase, by measuring the color, at different temperatures and pH 6.
Figure 4 shows the biostoning effect of EG28+CtCBD cellulase, by measuring the color, at different pH-values at 50°C.
Figure 5 shows the biostoning effect of EG28+CtCBD cellulase, by measuring the color, at different pH-values at 60°C.
Figure 6 shows the biostoning effect of EG28+CtCBD cellulase, by measuring the color, at different temperatures (pH 6).
Figure 7 shows the Influence of the pelleting temperature on beta-glucanase activity recovery of feeds supplemented with EG28 cellulase.
Figure 8 shows the Influence of the pelleting temperature on cellulase activity recovery of feeds supplemented with EG28+CtCBD cellulase.

### Detailed Description of the Invention

Cellulases comprise a catalytic domain/core (CD) expressing cellulase activity. In addition to the catalytic domain the cellulase molecule may comprise one or more "cellulose binding domains" ("CBDs"), also named as carbohydrate binding domains/modules (CBD/CBM), which can be located either at the N- or C-terminus of the catalytic domain. CBDs have carbohydrate-binding activity and they mediate the binding of the cellulase to crystalline cellulose but have little or no effect on hydrolytic activity of the enzyme on soluble substrates. These two domains are typically connected via a flexible and highly glycosylated linker region, herein called "linker". Such constructs are described e.g. in Srisodsuk et al., 1993. Some of the naturally occurring endoglucanases and cellobiohydrolases have a cellulose binding domain (CBD), while others do not.

Endoglucanases (EGs) are one of the three types of cellulases generally needed for the biological conversion of cellulose to glucose. Endoglucanases cut internal beta-1,4-glucosidic bonds, whereas cellobiohydrolases cut the disaccharide cellobiose from the end of the cellulose polymer chain and beta-1,4-glucosidases hydrolyze the cellobiose and other short cello-oligosaccharides to glucose. "Endoglucanase" ("EG") in connection with the present invention refers to enzymes classified as E.C. 3.2.1.4. They are 1,4-beta-D-glucan 4-glucanohydrolases and catalyze endohydrolysis of 1,4-beta-D-glycosidic linkages in polymers of glucose such as cellulose. Some endoglucanases may also hydrolyse e.g. 1,4-linkages in beta-D-glucans also containing 1,3-linkages. They may therefore also be classified as endo-1,3(4)-beta-glucanases (E.C. 3.2.1.6). Thus, an enzyme may catalyze reactions on several substrates and can belong to multiple classes.

Cellulases including endoglucanases can also be classified into various glycosyl hydrolase families according their primary sequence, supported by analysis of the three dimensional structure of some members of the family (Henrissat 1991, Henrissat and Bairoch 1993, 1996). For example family 45 (formerly celK) contains endoglucanases (EC 3.2.1.4), and family 5 (formerly known as celA) consists mainly of endoglucanases (EC 3.2.1.4). Family 7 (formerly cellulase family celC) contains both endoglucanases and cellobiohydrolases. Some glycosyl hydrolases are multifunctional enzymes that contain catalytic domains that belong to different glycosyl hydrolase families. For the present purposes the endoglucanase part of the fusion protein perferably belongs to the glycoside hydrolase family 45 or family 5, and more preferably to family 5.

According to a preferred embodiment of the invention the endoglucanase part of the fusion protein is derived from a fungus, preferably of the genus *Thermoascus*, and more preferably from *Thermoascus aurantiacus.* Such an endoglucanase has been described e.g. by Hong et al. 2003. WO03/062409 suggests using this enzyme for feed applications, because in addition to endoglucanase it also has beta-glucanase activity. Most preferably the endoglucanase is derived from *T*. *aurantiacus* strain ALKO4242 deposited as CBS 116239. The endoglucanase gene of said strain has been inserted into plasmid pALK1926 and deposited as DSM 17326. The protein encoded by this gene is herein referred to as "Ta EG28" or simply "EG28".

As reference endoglucanase part of the fusion protein may be obtained from *Acremonium sp.* preferably from *A. thermophilum,* and more preferably from a strain having the characteristics of the strain ALKO4245 deposited as CBS 116240. The endoglucanase obtainable from this strain and encoded by the gene *cel*45A is herein referred to as "At EG40" or simply "EG40".

Endoglucanase "core" as used herein, means the catalytic domain/core (CD) of an enzyme expressing at least endoglucanase activity. Such a catalytic domain may be in its naturally occurring form (i.e. intact) or, it may be modified.

According to one embodiment of the invention, the endoglucanase core has at least 75, 80, 85, 90, 95, 98 or 99% identity to SEQ ID NO: 2 (Ta EG28). Prefereably the core comprises at least the mature protein, which corresponds to amino acids 19 to 334 of SEQ ID NO: 2. The prediction on the signal sequence was made using the program SignalP V3.0 (Nielsen *et al.,* 1997; Bendtsen *et al.,* 2004); the NN value was obtained using neural networks and HMM value using hidden Markov models.

"Cellobiohydrolase" or "CBH" as used herein refers to enzymes that cleave cellulose from the end of the glucose chain and produce mainly cellobiose. They are also called 1,4-beta-D-glucan cellobiohydrolases or cellulose 1,4-beta-cellobiosidases. They hydrolyze the 1,4-beta-D-glucosidic linkages from the reducing or non-reducing ends of a polymer containing said linkages, such as cellulose, whereby cellobiose is released.

The CBD including the linker is preferably derived from *Chaetomium thermophilum,* and especially from the cellobiohydrolase (CBHI/Cel7A) encoding gene of strain ALK04265, deposited as CBS 730.95. This CBD including the linker is referred to as "CtCBD". According to a preferred embodiment of the invention, the linker plus the cellulose binding domain has a sequence that has at least 80, 85, 90, 95, 98 or 99% identity to SEQ ID NO: 15, (which corresponds to amino acids 335-415 of SEQ ID NO: 4). According to another preferred embodiment the second amino acid sequence comprises amino acids 335 - 379 of SEQ ID NO: 4.

The term "derived from" in connection with a microorganism source means that the polypeptide may naturally be produced by said specific microorganism source, or the polynucleotide encoding the polypeptide maybe isolated from said microoorganism source, or the term means a host cell into which the polynucleotide from said microorganism source encoding the polypeptide has been introduced. However it does not exclude minor modifications of the sequence e.g. by substitution, deletion, inversion and/or insertion of one or a few amino acids/codons as long as the biological activity of the encoded protein is retained.

The core, and the linker+CBD, respectively, may also be a fragment of said sequences, wherein said fragment has cellulase activity and/or cellulose binding activity. For example the first amino acid sequence may comprise a fragment of an amino acid sequence having at least 75% identity to SEQ ID NO: 2 or 8, and the second amino acid sequence may comprise a fragment of an amino acid sequence having at least 75% identity to SEQ ID NO: 15.

In the present context "cellulase activity" means catalytic ability to hydrolyse cellulose or derivatives thereof, such as endoglucanase or beta-glucanase activity. In addition to endoglucanase and/or beta-glucanase activity, some of the cellulases may further have hemicellulase and/or xylanase activity.

As used in the present context the term "identity" refers to the global identity between two amino acid sequences compared to each other from the first amino acid encoded by the corresponding gene to the last amino acid. The identity of the full-length sequences is measured by using Needleman-Wunsch global alignment program at EMBOSS (European Molecular Biology Open Software Suite; Rice et al., 2000) program package, version 3.0.0, with the following parameters: EMBLOSUM62, Gap penalty 10.0, Extend penalty 0.5. The algorithm is described in Needleman and Wunsch (1970). The man skilled in the art is aware of the fact that results using Needleman-Wunsch algorithm are comparative only when aligning corresponding domains of the sequence. Consequently comparison of e.g. cellulase sequences including CBD or signal sequences with sequences lacking those elements are excluded as not being meaningful.

According to one embodiment of the invention the fusion protein contains an endoglucanase core encoded by a gene similar to that included in *E*. *coli* DSM 17326. Preferebly the fusion protein is encoded by a fusion gene similar to that included in *E. coli* DSM 18159. According to a specific embodiment of the invention the fusion protein contains an endoglucanase core comprising the sequence of SEQ ID NO:2 and a linker and CBD comprising the sequence of SEQ ID NO: 15. Especially it comprises the amino acid sequence of SEQ ID NO: 4, or a fragment thereof having cellulase and cellulose binding activity.

A "fragment" is understood to be part of a specific amino acid sequence that is long enough to have the desired biological activity. In other words the fragment may be e.g. only the mature part of the amino acid sequence or even a subsequence of the mature part. Biological activity in this context thus refers to cellulase activity, cellulose binding activity or both.

The fusion protein of the invention may be prepared by attaching the endoglucanase core part to the linker and CBD part in the appropriate encoding DNA using generally known recombinant DNA techniques for producing the desired recombinant protein. Briefly the polynucleotides encoding the fusion partners are amplified and cloned, nucleotides may also be synthetized. The fused polynucleotide is inserted into an expression vector, transformed into a host cell and expressed. Preferably the linker and CBD is attached at the C-terminus of the endoglucanase core.

An "expression vector" is a cloning plasmid or vector capable of expressing DNA encoding the endoglucanase fusion proteins after transformation into a desired host. When a fungal host is used, the gene of interest is preferably provided to a fungal host as part of a cloning or expression vehicle that integrates into the fungal chromosome, or allows the gene of interest to integrate into the host chromosome. Other sequences that are part of the cloning vehicle or expression vehicle may also be integrated with said DNA during the integration process. In addition, in fungi the expression vector or parts thereof can be targeted into predetermined loci. Alternatively, the desired fusion gene is provided as an autonomously replicating plasmid.

The DNA encoding the endoglucanase fusion proteins is preferably placed under the control of (i.e., operably linked to) certain control sequences such as promoter sequences provided by the vector. Upon transformation these control sequences integrate into the host genome with the gene of interest. Alternatively, the control sequences can be those at the integration site.

The expression control sequences of an expression vector will vary depending on whether the vector is designed to express a certain gene in a prokaryotic or in a eukaryotic host (for example, a shuttle vector may provide a gene for selection in bacterial hosts). Expression control sequences can contain transcriptional regulatory elements such as promoters, enhancer elements, and transcriptional termination sequences, and/or translational regulatory elements, such as translational initiation and termination sites.

A polynucleotide molecule, such as DNA, is said to be capable of expressing a polypeptide, if it contains expression control sequences, which contain transcriptional regulatory information and such sequences are operably linked to the nucleotide sequence, which encodes the polypeptide.

An operable linkage is a linkage in which a sequence is connected to a regulatory sequence (or sequences) in such a way as to place expression of the sequence under the influence or control of the regulatory sequence. Two DNA sequences (such as a promoter region sequence linked to the 5' end of the protein encoding sequence) are said to be operably linked if function of promoter results in the transcription.

The vectors of the invention may further comprise other operably linked regulatory elements, such as enhancer sequences.

In a preferred embodiment, genetically stable transformants are constructed, whereby the DNA encoding the fusion proteins is integrated into the host chromosome by transformation with a vector, which may harbor sequences promoting integration of said vector into the chromosome.

Cells that have stably integrated DNA encoding the endoglucanase fusion proteins into their chromosomes may be selected e.g. by introduced marker(s), homologous or heterologous, which allow for selection of host cells which contain the expression vector in the chromosome, for example the marker may provide biocide resistance, e.g., resistance to antibiotics, or heavy metals, such as copper, or markers complementing an auxotrophic mutation in the host chromosome, and the like. The selectable marker can for example be a selection gene directly linked to the DNA gene sequences to be expressed, or introduced into the same cell by co-transformation. Also other selection systems can be used.

Once the expression vector containing the DNA encoding the fusion protein is prepared, it is introduced into an appropriate host cell by any of a variety of suitable means, including transformation as known in the art. After transformation, recipient cells are grown in an appropriate selective medium, which selects for the growth of transformed cells.

Suitable expression and production host systems are for example the production system developed for fungal hosts *Trichoderma* (EP 244 234), or *Aspergillus,* such as *A. oryzae* or *A. niger* (WO 97/08325 and WO 95/33386, U.S. Patent No. 5,843,745, U.S. Patent No. 5,770,418), or the production system developed for *Fusarium,* such as *F. oxysporum* (Malardier et al., 1989). Suitable production systems developed for bacteria include a production system developed for *Bacillus,* for example *B. subtilis, B.licheniformis, B. amyloliquefaciens* or for *E*. *coli,* or for an actinomycete *Streptomyces.* Suitable production systems developed for yeasts are systems developed for *Saccharo-myces, Shizosaccharomyces* or *Pichia pastoris.* Production systems in other microbes or in mammalian cells or in plants are also possible.

Expression of the cloned gene sequence(s) results in the production of the desired protein, or in the production of a fragment of this protein. This expression can take place in a continuous manner in the transformed cells, or in a controlled manner.

To obtain the enzyme preparations of the invention, the hosts having the desired properties (that is, hosts capable of expressing economically feasible quantities of the endoglucanase fusion proteins) are cultivated under suitable conditions, and the desired enzymes are preferably secreted from the hosts into the culture medium, and optionally recovered from said culture medium by methods known in the art. Preferably the host for such production is a filamentous fungus, such as *Trichoderma or Aspergillus,* and especially *T*. *reesei.*

As used in the present context the "enzyme preparation" refers to any enzyme product, which contains at least one endoglucanase fusion protein of the invention. Thus, such an enzyme preparation may be a spent culture medium or filtrate. Spent culture medium means the culture medium of the host comprising the produced enzymes. Preferably the host cells are separated from said medium after the production. If desired, such preparations may be lyophilized or the enzymatic activity otherwise concentrated and/or stabilized for storage. If required, a desired enzyme may be further purified in accordance with conventional methods, such as extraction, precipitation, chromatography, affinity chromatography, electrophoresis, or the like.

However, it is an advantage of the invention that the culture medium with or without host cells may be utilized as an enzyme preparation as such without further purification, because the endoglucanase fusion proteins can be secreted into the culture medium, and they display activity in the ambient conditions of the spent culture medium. The enzyme preparations are very economical to provide and use, because isolation of a specific enzyme from the culture medium is unnecessary.

In addition to the endoglucanase fusion protein, the enzyme preparations may comprise one or more other enzymes, which may be for example other cellulases, amylases, lipases, proteases, hemicellulases, xylanases, pectinases and/or oxidases such as laccases and peroxidases. Alternatively, before, during or after the treatment with the endoglucanase fusion protein another enzyme treatment may be carried out. The enzyme treatment may comprise, for example, one or more amylase treatments (e.g. for desizing of denim), one or more cellulase treatments and/or one or more peroxidase and/or laccase treatments. It depends on the application what other enzymes are included in the enzyme preparation or used in the enzyme treatment.

In addition to the fusion protein, the enzyme preparation may contain additives, such as stabilizers, buffers, preservatives, surfactants and/or culture medium components. Preferred additives are such, which are commonly used in enzyme preparations intended for the application, where the enzyme preparation is used.

The enzyme preparations may be provided as a liquid or as a solid, for example, as a dried powder or granular, especially non-dusting granules, or a stabilized liquid. It is envisioned that the enzyme preparations can be further enriched, or made partially or completely deficient in specific enzymatic activities, so as to satisfy the requirements of a specific utility in various applications e.g. in the textile industry. A mixture of enzyme activities secreted by a host can be advantageous in a particular industrial application, for example in biofinishing and biostoning.

The endoglucanase fusion proteins and the preparations thereof are useful e.g. in textile, feed, plant oil, detergent, and pulp and paper industry. They may be used for treating any cellulosic material, such as textile material, plants used in animal feed, plant material for oil extraction, or wood-derived mechanical or chemical pulp or secondary fiber. They may also be added into detergents, which normally contain auxiliaries, such as surface-active agents, surfactants, bleaching agents and/or builders. In the present context "cellulosic material" refers to any material comprising cellulose or derivatives thereof as a significant component. The cellulosic material is contacted with an effective amount of the fusion protein under suitable conditions, such as appropriate pH, and temperature, and the reaction is allowed to continue for a time sufficient for the enzymatic reaction to take place.

The fusion enzymes are especially useful in the treatment of textile materials, such as fabrics and garments. The textile material may be manufactured of natural cellulose containing fibers or man-made cellulose containing fibers or mixtures thereof, or a blend of synthetic fibers and cellulose containing fibers. Preferably the cellulose containing material is cotton, especially denim. By "denim" is meant, in connection of this invention, denim fabric, usually denim garments, particularly jeans. Advantageously the denim is Indigo dyed denim. Denim can also be treated with derivatives of Indigo or with Indigo together with some other dye, for example Indigo-dyed denim with sulphur bottom.

The fusion endoglucanases are especially useful in biostoning and biofinishing of textiles.

Stone washing has three steps: desizing, abrasion and after-treatment. The first step, the desizing process is normally the first wet treatment of jeans and means removal of starch or other sizing agents usually applied to the warp yarns to prevent damage during the weaving process. Alpha-amylases are used to remove starch-based sizing agents for improved and uniform wet processing. After desizing the jeans are normally rinsed with water or passed directly to the abrasion step.

The second step, abrasion, can be performed with enzymes or pumice stones or both. In all cases mechanical action is needed to remove the dye, and the treatment is usually carried out in washing machines, like drum washers. The term "abraded" means the appearance of denim fabric, when it has been treated by cellulase enzymes or stones, or both. Synonymous expressions are "stone washed look" or "worn look". As a result of uneven dye removal there are contrasts between dyed areas and areas from which dye has been removed.

Abrasion is generally followed by the third step, after-treatment that includes washing and rinsing steps during which detergents, optical brighteners, bleaching agents or softeners may be used. After the enzymatic treatment the reaction must be stopped in order to prevent damage of the treated materials, for example by temperature and/or pH inactivation, the latter comprising a thorough rinsing and/or detergent wash-off. This ensures that the mechanical strength of the fiber is not further compromised by the continued presence of the enzyme.

As used in the present context the expression "biostoning" of fabric or garment means the use of enzymes in place of, or in addition to, pumice stones for the treatment of fabric or garment, especially denim.

As stated above, treatment with cellulase can completely replace treatment with pumice stones (for example, 1 kg commercial enzyme vs. 100 kg stones). However, cellulase treatment can also be combined with pumice stone treatment, when it is desired to produce a heavily abraded finish. A peach skin effect in which a fine protruding hair-like covering is created is also achieved by a wash combining a neutral cellulase with pumice stones. The fusion proteins described are especially useful to efficiently provide an abraded look and to minimize backstaining in biostoning.

Biostoning is typically performed at about pH 3.0 to 8.0, and preferably at pH 5.0 to 7.0. The temperature of the reaction can range from about 30°C to 80°C and is preferably between 50 to 60°C. The liquor ratio (the ratio of the volume of liquid per weight of fabric) may range from about 3:1 to 20:1, preferably 5:1 to 10:1. The treatment time can range between 15 min to 90 min and preferably 30 min to 60 min. It should be emphasized that the enzyme dosage greatly depends on the type of the fabrics, machinery, process conditions (pH, temperature, liquor ratio, treatment time, denim load, process scale) and type of enzyme preparation and like. If desired, pumice stones can be used in combination with the endoglucanase fusion proteins. The enzyme dosage required will then be significantly lower. A person skilled in art is capable in defining suitable dosages and conditions.

The endoglucanase fusion proteins of the present invention provide unexpected advantages in that the enzyme performance is high and the back-staining is low resulting in very good contrast. In addition the fusion proteins are easily manufactured, and they may be used in a relatively broad range of temperature and pH.

Further, the endoglucanase fusion proteins are useful in biofinishing of fabrics and garments. "Biofinishing" refers to the use of enzymes in a controlled hydrolysis of cellulosic fibers in order to modify the fabric or yarn surface in a manner that prevents permanently pilling, improves fabric handle like softness and smoothness, clears the surface structure by reducing fuzzing, which results in clarification of colors, improves the drapability of the fabric, improves moisture absorbency and which may improve also the dyeability.

Enzymatic depilling can be carried out at any stage during textile wet processing, preferably after optional desizing and/or bleaching, and similar conditions as in biostoning can be used.

The endoglucanase fusion proteins possessing also beta-glucanase, hemicellulase or xylanase activity are further useful for improving the quality of animal feed, whereby plant material is treated with the enzymes.

Starch, proteins and lipids can be easily degraded by the digestive system of monogastric animals such as poultry and pigs, whereas the major part of non starch polysaccharides (NSP) including mixed-linked beta-glucans of e.g. barley and oats remain intact due to the lack of such enzyme activities within the animal. Furthermore, the digestibility of other components, particularly animal-based fats, is reduced in the presence of NSP.

Beta-glucanases have been commercially used to alleviate problems caused by mixed-linked beta-glucans of barley and oats. Beta-glucanases are known to reduce intestinal viscosity caused by soluble beta-glucans as well as to release nutrients encapsulated by cell-walls rich in beta-glucans. The use of beta-glucanases improves animal performance, which can be seen as improved weight gain and feed conversion ratio. Also, incidences of sticky droppings in poultry are typically reduced.

Steam pelleting is the main feed processing technology throughout the world. Its advantages over production of mash feeds include easier handling, decrease of toxic substances and organisms, and above all improved feed efficiency. The thermotolerance and high performance of the fusion proteins described herein make them suitable for feed applications.

The invention is illustrated by the following non-limiting examples. It should be understood, however, that the embodiments given in the description above and in the examples are for illustrative purposes only, and that various changes and modifications are possible within the scope of the invention.

### Example 1. Production of Thermoascus aurantiacus ALK04242 EG28 cellulase in Trichoderma reesei

Standard molecular biology methods were used in the isolation and enzyme treatments of DNA (plasmids, DNA fragments), in *E. coli* transformations, etc. The basic methods used are described in the standard molecular biology handbooks, e.g. Sambrook et al. (1989) and Sambrook and Russell (2001).

The *Thermoascus aurantiacus cel*5A gene (SEQ ID NO: 1) encoding EG28 cellulase (SEQ ID NO: 2) was amplified by PCR directly from the *T*. *aurantiacus* ALKO4242 genomic DNA, isolated by the method of Raeder and Broda (1985). The forward (SEQ ID NO: 9) and reverse (SEQ ID NO: 10) primers were designed on the basis of the published *T. aurantiacus* endoglucanase sequence (AF487830). The amplified 1.3 kb product containing the exact gene (from START to STOP codon) was cloned as a *Sac*II-*Pst*I fragment into the pBluescript II KS+ vector. Two independent clones were sequenced and one clone was selected and designated as pALK1926. The deposit number of the *E*. *coli* strain containing pALK1926 in the Deutsche Sammlung von Mikroorganismen und Zellkulturen GmbH culture collection is DSM 17326.

An expression plasmid (pALK1930, Fig. 1) was constructed for production of recombinant *T. aurantiacus* cellulase EG28/Cel5A (SEQ ID NO: 2). The *cel*5A gene, including its own signal sequence, was exactly fused to the *T*. *reesei cbh1 (cel7A)* promoter by PCR. The *cbh1* promoter, *cbh1* terminator and *amdS* marker gene were included as described in Paloheimo *et al.* (2003). The linear expression cassette (Fig. 1) was isolated from the vector backbone by restriction enzyme digestion, transformed into T. *reesei* A96, and transformants were selected with acetamide as sole nitrogen source. The host strain lacks four major endogenous cellulases: CBHI/Cel7A, CBHII/Cel6A, EGI/Cel7B and EGII/Cel5A. Transformations were performed according to Penttilä *et al.* (1987) with the modifications described in Karhunen *et al.* (1993). The transformants were purified on selection plates through single conidia prior to sporulating them on PD.

The cellulase production of the transformants was analyzed from the culture supernatants of shake flask cultivations (50 ml). Transformants were grown for 7 days in a complex cellulose-inducing medium (Joutsjoki *et al.* 1993) buffered with 5% KH₂PO₄ at pH 5.5. The enzyme activity of the recombinant protein was measured from the culture supernatant as the release of reducing sugars from carboxymethylcellulose (2% CMC) at 50°C in 50 mM Sitrate buffer pH 4.8 essentially as described by Bailey and Nevalainen 1981; Haakana *et al.* 2004. Activity against barley beta-glucan (1%) was also determined by measuring the release of reducing sugars at 50°C in 50 mM acetate buffer pH 4.8 as described by Stålbrand *et al.* 1993. Production of the recombinant protein was also detected from the culture supernatant by SDS-polyacrylamide gel electrophoresis. The genotypes of the chosen transformants were analysed by Southern blotting using the expression cassette as a probe.

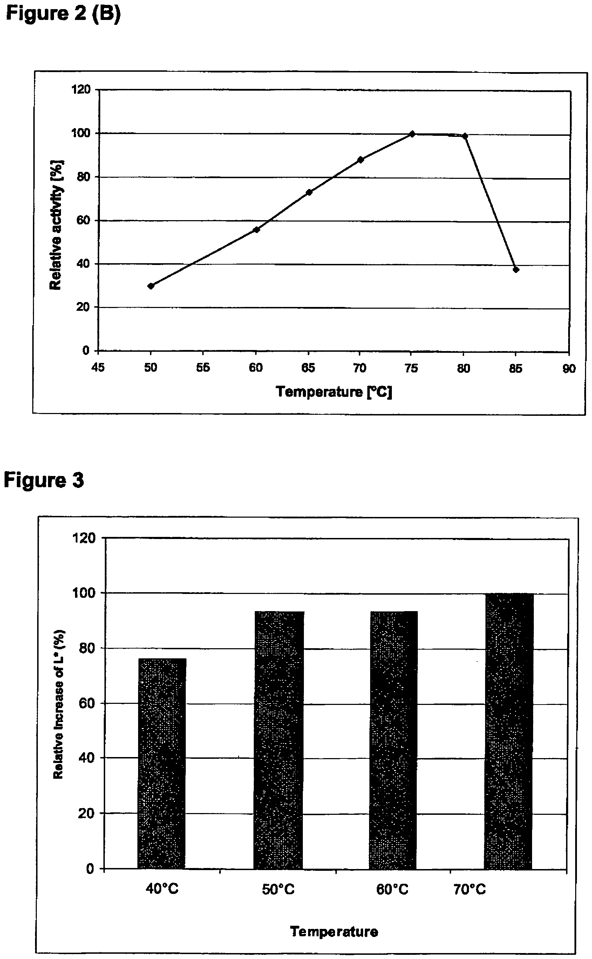

The pH optimum of the heterologously produced Ta EG28/Cel5A cellulase was determined in the universal Mcllvaine's buffer within a pH range of 4.0 to 8.0 using carboxymethylcellulose as substrate. As shown in Figure 2A the pH optimum for EG28/Cel5A cellulase activity is at pH 6.0. The optimal temperature for the enzymatic activity of EG28/Cel5A cellulase was determined to be 75°C (Figure 2B).

The chosen transformant (RF6188) was cultivated in a bioreactor to obtain material for the application tests (see Example 4).

### Example 2. Production of the recombinant Thermoascus aurantiacus ALK04242 EG28+CtCBD fusion protein

To produce a recombinant *Thermoascus aurantiacus* EG28+CtCBD fusion protein (SEQ ID NO: 4), the cellulose binding domain (CBD) of the CBHI/Cel7A cellulase of *Chaetomium thermophilum* ALKO4265 was linked to the EG28/Cel5A cellulase. The construct contains the catalytic domain of EG28 (amino acids 1-334 of the full-length polypeptide) attached to the linker region and CBD of *C*. *thermophilum* CBHI/ Cel7A CtCBD (SEQ ID NO: 15).

Standard molecular biology methods were used as described in Example 1. First, a unique *Sna*BI restriction site near the C-terminal end of the EG28/Cel5A sequence was introduced by PCR. This enables direct fusion of any blunt-ended DNA after amino acid Y334 of the EG28/Cel5A polypeptide. The linker and CBD region of the *C*. *thermophilum* CBHI/Cel7A encoding gene (*cel*7A, SEQ ID NO: 7) was amplified by PCR using forward (SEQ ID NO: 11) and reverse (SEQ ID NO: 12) primers and C. *thermophilum* ALK04265 genomic DNA as template. The amplified 1.6 kb product was ligated to the *cel*5A gene (after Y334) to create Ta *cel*5A_Ct *cel*7AlinkerCBD (SEQ ID NO: 3). The resulting plasmid was designated as pALK1946. The deposit number of the *E*. *coli* strain containing pALK1946 in the Deutsche Sammlung von Mikroorganismen und Zellkulturen GmbH culture collection is DSM 18159.

An expression plasmid (pALK1948, Fig 1) for production of the EG28+CtCBD cellulase was constructed as described in Example 1. The linear 8.9 kb expression cassette (Figure 1) was isolated from the vector backbone by *Not*I restriction enzyme digestion, transformed into *T*. *reesei* A33 (the strain has the genes encoding the four major cellulases CBHI/Cel7A, CBHII/Cel6A, EGI/Cel7B and EGII/Cel5A deleted), and transformants selected as described in Example 1. The pH and temperature optimum of the fusion protein was determined to be the same as those of the wild-type EG28 protein.

The chosen transformant (RF6377) was cultivated in a bioreactor to obtain material for the application tests (see Examples 5 to 10).

### Example 3 Reference. Production of the recombinant Acremonium thermophilum ALK04245 EG40+CtCBD fusion protein

For production of a recombinant *Acremonium thermophilum* ALK04245 EG40+CtCBD fusion protein (SEQ ID NO: 6), the cellulose binding domain of EG40 cellulase is replaced with that of the *Chaetomium thermophilum* ALKO4265 CBHI/Cel7A. The construct contains the catalytic domain of EG40 cellulase (amino acids 1 to 234 of the fult-length polypeptide) attached to the linker region and CBD of *C*. *thermophilum* CBHI/Cel7A (CtCBD, SEQ ID NO: 15).

Standard molecular biology methods are used as described in Example 1. The *Acremonium thermophilum cel*45A gene (SEQ ID NO: 8) is amplified by PCR from the *A. thermophilum* ALKO4245 genomic DNA using primers (SEQ ID NO: 13) and (SEQ ID NO: 14). The 1.1 kb PCR fragment is cloned as a *Sac*II-*Pst*I fragment into the pBluescript II KS+ vector. After this, a unique *Nru*I restriction site near the C-terminal end of the EG40 sequence is introduced by PCR. This enables direct fusion of any blunt-ended DNA after amino acid S234 of the EG40 polypeptide. The linker plus CBD region of the CBHI/Cel7A cellulase of *Chaetomium thermophilum* (*cel*7A) is amplified by PCR as described in Example 2 and a restriction fragment thereof ligated to the *cel*45A gene (after S234) to create At *cel*45A_Ct *cel*7AlinkerCBD (SEQ ID NO: 5). An expression plasmid for production of the EG40+CtCBD cellulase is constructed and the recombinant protein (SEQ ID NO: 6) is produced in *Trichoderma* as described in Example 1.

### Example 4. Performance of EG28 cellulase in denim finishing at different temperatures

*Thermoascus aurantiacus* EG28 cellulase produced in *Trichoderma* (strain RF6188) as described in Example 1 was tested for its ability to create an abraded look similar to that provided by pumice stones in biostoning of denim at different temperatures. An efficient denim finishing enzyme, a commercial EGII enriched acid cellulase preparation produced in *Trichoderma* (US. 5,874,293) was used as a reference.

Jeans made of Indigo dyed denim twill were used as test material after desizing with ECOSTONE^{®} A200 alpha-amylase. The cellulase treatments were performed with Electrolux's Wascator FOM 71 CLS washer extractor under conditions described in Table 1.

The endoglucanase activity (ECU) of the enzyme preparations used was measured as the release of reducing sugars from hydroxyethyl cellulose as previously described (Bailey and Nevalainen, 1981). The EGII enriched enzyme concentrate and EG28 preparation were dosed at 220 ECU/g and ca. 380 ECU/g fabric, respectively. The enzymes were tested at their optimal pH range, the EGII preparation at pH 5 and the EG28 preparation at pH 6. After draining, the enzyme was inactivated (for 10 minutes at 40°C) by raising the pH above 11 with sodium hydroxide. The jeans were then rinsed three times with water and dried in a tumbler.

The biostoning effect/abrasion level was evaluated by measuring the color as reflectance values with a Minolta CM 2500 spectrophotometer using L*a*b* color space cooridinates (illuminant D65/ 2°). The color from the face and the reverse side of denim and of the pockets was measured after desizing (i.e. before cellulase treatment) and after the cellulase treatment. Each measurement on the face side, reverse side or pockets was the average of approximate 40, 20, or 12 measurements, respectively. Two pairs of jeans were used in each test and the final result was the average thereof. The results are shown in Table 2 and Figure 3.

**Table 1. Test conditions/process parameters used in cellulase treatments.**

| **Process parameter** | |
|---|---|
| Denim load | 1.3 kg |
| Water | 19 liter |
| pH control (pH 5 - 5.3) | 35 g Na₂HPO₄2H₂O + 22 g citric acid |
| pH control (pH 6.1 - 6.3) | 35.5 g Na₂HPO₄2H₂O + 15 g citric acid |
| Time | 45 min |
| Temperature | 40, 50, 60 or 70°C |
| Cellulase dosage | 220 or ca. 380 ECU/g fabric |

**Table 2. Color measurements of denim treated with EG28 cellulase at different temperatures.**

| Treatment with EGII enriched preparation was used as reference. L* indicates the lightness, -b* is the blue direction, +b* is the yellow direction. | | | | | | | | |
|---|---|---|---|---|---|---|---|---|
| **Enzyme preparation** | **ECU/g garment** | **Conditions** | **Before cellulase Treatment** | | **After cellulase Treatment** | | **deltaL*** | **deltab*** |
| | | | **L*** | **b*** | **L*** | **b*** | | |
| **Face side:** | | | | | | | | |
| EG28 | 380 | 70°C, pH 6.1 - 6.3 | 23.67 | -16.07 | **32.63** | -16.94 | **8.96** | -0.87 |
| EG28 | 370 | 60°C, pH 6.1 - 6.3 | 23.67 | -16.38 | 31.94 | -17.09 | **8.27** | -0.71 |
| EG28 | 380 | 50°C, pH 6.1 - 6.3 | 23.62 | -16.24 | 31.91 | -16.86 | **8.30** | -0.63 |
| EG28 | 380 | 40°C, pH 6.1 - 6.3 | 23.75 | -16.07 | 30.57 | -17.32 | **6.82** | -1.25 |
| EGII enriched | 220 | 60°C, pH 5.2 - 5.3 | 23.45 | -16.10 | **32.90** | -17.29 | **9.46** | -1.19 |
| **Reverse side:** | | | | | | | | |
| EG28 | 380 | 70°C, pH 6.1 - 6.3 | 49.76 | -6.95 | **49.60** | **-10.43** | **-0.16** | **-3.48** |
| EG28 | 370 | 60°C, pH 6.1 - 6.3 | 49.55 | -6.87 | 50.78 | -9.57 | **1.24** | -2.71 |
| EG28 | 380 | 50°C, pH 6.1 - 6.3 | 49.92 | -7.05 | 50.49 | -9.83 | **0.57** | -2.78 |
| EG28 | 380 | 40°C, pH 6.1 - 6.3 | 49.89 | -6.93 | 49.73 | -9.73 | **-0.16** | -2.80 |
| EGII enriched | 220 | 60°C, pH 5.2 -5.3 | 49.75 | -6.82 | **48.28** | **-12.26** | **-1.47** | **-5.45** |
| **Pockets:** | | | | | | | | |
| EG28 | 380 | 70°C, pH 6.1 - 6.3 | 77.40 | -8.14 | **68.53** | **-12.60** | **-8.86** | **-4.47** |
| EG28 | 374 | 60°C, pH 6.1 - 6.3 | 77.91 | -7.71 | 70.29 | -12.34 | **-7.62** | -4.63 |
| EG28 | 380 | 50°C, pH 6.1 - 6.3 | 78.11 | -7.62 | 70.63 | -12.02 | **-7.49** | -4.40 |
| EG28 | 380 | 40°C, pH 6.1 - 6.3 | 77.51 | -7.60 | 70.47 | -11.91 | **-7.04** | -4.32 |
| EGII enriched | 220 | 60°C, pH 5.2 -5.3 | 77.46 | -7.68 | **66.18** | **-14.17** | **-11.29** | **-6.49** |

The results in Table 2 and Fig. 3 show that the best abrasion effect with the EG28 preparation (strain RF6188) was obtained at the range of 50 to 70°C. Using a dosage of 380 ECU/g fabric of the EG28 preparation at 70°C a similar abrasion level (lightness L*) was obtained compared to using the dosage of 220 ECU/g fabric of an EGII enriched preparation at 60°C. However, the back-staining effect (re-deposition of Indigo-dye) on the reverse side of the denim and pockets was lower with the EG28 preparation (higher lightness, less blue) than with the EGII enriched preparation. Also the contrast on the face side of the denim looked better.

### Example 5. Performance of EG28+CtCBD fusion protein compared to EG28 cellulase in denim finishing

Recombinant *Thermoascus aurantiacus* EG28+CtCBD fusion protein produced in *Trichoderma* (strain RF6377) as described in Example 2 was compared to the EG28 preparation from strain RF6188 in biostoning of denim at pH 6, 60°C. The denim and test system for biostoning were as in Example 4, except that the amount of denim was leveled to 1430 g with an extra piece of different denim that was not included in the measurements. The effect of the cellulase treatment was evaluated as in Example 4.

The results in Table 3 show that the biostoning effect of the EG28+CtCBD preparation was very good at a low dosage. With strain RF6377 a similar abrasion level (lightness **L***) was obtained with 6.5 times lower dosage compared to RF6188. Linking of cellulose binding domain (CBD) to EG28 cellulase did not increase the undesirable backstaining effect, but increased the desired washing performance greatly.

**Table 3. Color measurements of denim treated with EG28 and EG 28+CtCBD cellulases at 60 °C, pH 6.**

| L* indicates the lightness, -b* is the blue direction, +b* is the yellow direction. | | | | | | | |
|---|---|---|---|---|---|---|---|
| **Enzyme preparation** | **ECU/g garment** | **Before cellulase treatment** | | **After cellulase Treatment** | | **deltaL*** | **deltab*** |
| | | **L*** | **b*** | **L*** | **b*** | | |
| **Face side:** | | | | | | | |
| EG28 | 325 | 23.40 | -16.02 | **31.01** | -17.12 | **7.62** | -1.10 |
| EG28+CtCBD | 50 | 23.81 | -16.21 | **31.60** | -16.92 | **7.80** | -0.71 |
| **Reverse side:** | | | | | | | |
| EG28 | 325 | 49.46 | -6.83 | **50.03** | -9.62 | **0.57** | -2.79 |
| EG28+CtCBD | 50 | 49.36 | -7.03 | **50.62** | -9.61 | **1.27** | -2.58 |
| **Pockets:** | | | | | | | |
| EG28 | 325 | 76.11 | -8.36 | **68.53** | -12.46 | **-7.58** | -4.10 |
| EG28+CtCBD | 50 | 76.02 | -8.45 | **68.81** | -12.39 | **-7.21** | -3.94 |

### Example 6. Performance of EG28+CtCBD cellulase compared to EGII cellulase in denim finishing

Recombinant *Thermoascus aurantiacus* EG28+CtCBD fusion protein produced in *Trichoderma* (strain RF6377) was compared to the commercial EGII enriched acid cellulase preparation (US. 5,874,293) in biostoning of denim.

The test system for biostoning was as in Example 4, except that pieces (legs) of several different Denim types from Ukos Sport (Belgium) and Vicunha (Brazil) were used (total 1.2 kg). The conditions for EG28+CtCBD and EGII treatment were as in Table 4. The effect of the cellulase treatment was evaluated as in Example 4.

The results of the experiments performed with four different denim fabrics are shown in Tables 4 and 5. Using an enzyme dosage of 500 ECU/g fabric of EG28+CtCBD preparation a similar lightness level was obtained compared to using a dosage of 1000 ECU/g of the EGII enriched preparation. The performance of the EG28+CtCBD preparation required roughly only half of the ECU-activity of that of EGII enriched cellulase. Also the culture medium of EG28+CtCBD produced by the recombinant host is volumetrically about twofold as effective as that of the EGII producing recombinant host in biostoning. Considerably less backstaining was observed on the reverse side of the denim when EG28+CtCBD preparation was used. The performance of the EG28+CtCBD preparation at 50°C was better at pH 6 than at pH 5.

**Table 4. Color measurements on the face side of denim treated with EG28+CtCBD cellulase at pH 5 and 6. Treatment with EGII enriched preparation was used for comparison. L* indicates the lightness, -b* is the blue direction, +b* is the yellow direction.**

| **Enzyme preparation** | **Denim** | **ECU/g garment** | **Conditions** | **Before cellulase Treatment** | | **After cellulase treatment** | | **deltaL*** | **deltab*** |
|---|---|---|---|---|---|---|---|---|---|
| | | | | **L*** | **b*** | **L*** | **b*** | | |
| **EGII Enriched** | Atlanta, Ukos sport | 1000 | pH adj. with acetic acid 55 min, 50°C, pH 5 | 19.96 | -15.91 | 27.44 | -18.49 | 7.48 | -2.58 |
| | Mathew, Ukos sport | | | 18.28 | -7.97 | 21.87 | -11.71 | 3.59 | -3.74 |
| | Nostalgy, Ukos sport | | | 19.12 | -8.78 | 23.90 | -13.50 | 4.78 | -4.72 |
| | Vicunha, Savana | | | 17.24 | -10.49 | 21.51 | -14.93 | 4.27 | -4.44 |
| | **Average** | | | **18.65** | **-10.79** | **23.68** | **-14.66** | **5.03** | **-3.87** |
| **EG28+CtCBD** | Atlanta, Ukos sport | 500 | pH adj. with acetic acid 55 min, 50°C, pH 5 | 19.79 | -16.02 | 25.88 | -18.44 | 6.09 | -2.42 |
| | Mathew, Ukos sport | | | 18.07 | -8.58 | 21.45 | -11.80 | 3.38 | -3.22 |
| | Nostalgy, Ukos sport | | | 19.05 | -8.73 | 22.65 | -12.69 | 3.60 | -3.96 |
| | Vicunha, Savana | | | 17.38 | -10.11 | 20.75 | -14.18 | 3.37 | -4.07 |
| | **Average** | | | **18.57** | **-10.86** | **22.68** | **-14.28** | **4.11** | **-3.42** |
| **EG28+CtCBD** | Atlanta, Ukos sport | 500 | pH adj. with acetic acid 55 min, 50°C, pH 6 | 19.98 | -16.18 | 27.35 | -18.20 | 7.37 | -2.02 |
| | Mathew, Ukos sport | | | 17.51 | -8.84 | 21.40 | -11.62 | 3.89 | -2.78 |
| | Nostalgy, Ukos sport | | | 18.58 | -8.65 | 22.96 | -12.75 | 4.38 | -4.10 |
| | Vicunha, Savana | | | 17.50 | -10.16 | 21.49 | -14.06 | 3.99 | -3.90 |
| | **Average** | | | **18.39** | **-10.96** | **23.30** | **-14.16** | **4.91** | **-3.20** |

**Table 5. Color measurements on the reverse side of denim treated with EG28+CtCBD cellulase at pH 5 and 6. Treatment with EGII enriched preparation was used for comparison. L* indicates the lightness, -b* is the blue direction.**

| **Enzyme preparation** | **Denim** | **ECU/g garment** | **Conditions** | **Before cellulase treatment** | | **After cellulase treatment** | | **deltaL*** | **deltab*** |
|---|---|---|---|---|---|---|---|---|---|
| | | | | **L*** | **b*** | **L*** | **b*** | | |
| **EGII Enriched** | Atlanta, Ukos sport | 1000 | pH adj. with acetic acid 55 min, 50°C, pH 5 | 47.98 | -7.25 | 40.18 | -14.63 | -7.80 | -7.38 |
| | Mathew, Ukos sport | | | 38.41 | -7.17 | 36.11 | -10.93 | -2.30 | -3.76 |
| | Nostalgy, Ukos sport | | | 46.70 | -6.06 | 41.87 | -11.73 | -4.83 | -5.67 |
| | Vicunha, Savana | | | 35.65 | -9.24 | 33.48 | -12.44 | -2.17 | -3.20 |
| | **Average** | | | **42.19** | **-7.43** | **37.91** | **-12.43** | **-4.28** | **-5.00** |
| **EG28+CtCBD** | Atlanta, Ukos sport | 500 | pH adj. with acetic acid 55 min, 50°C, pH 5 | 48.01 | -7.29 | 41.82 | -13.25 | -6.19 | -5.96 |
| | Mathew, Ukos sport | | | 37.60 | -7.27 | 35.59 | -10.50 | -2.01 | -3.23 |
| | Nostalgy, Ukos sport | | | 46.79 | -5.99 | 44.75 | -9.37 | -2.04 | -3.38 |
| | Vicunha, Savana | | | 35.08 | -9.48 | 33.72 | -11.80 | -1.36 | -2.32 |
| | **Average** | | | **41.87** | **-7.51** | **38.97** | **-11.23** | **-2.90** | **-3.72** |
| **EG28+CtCBD** | Atlanta, Ukos sport | 500 | pH adj. with acetic acid 55 min, 50°C, pH 6 | 47.55 | -7.43 | 42.41 | -13.30 | -5.14 | -5.87 |
| | Mathew, Matthew, Ukos sport | | | 37.27 | -7.58 | 36.01 | -10.27 | -1.26 | -2.69 |
| | Nostalgy, Ukos sport | | | 47.02 | -6.01 | 44.61 | -10.13 | -2.41 | -4.12 |
| | Vicunha, Savana | | | 34.84 | -9.39 | 33.53 | -11.20 | -1.31 | -1.81 |
| | **Average** | | | **41.67** | **-7.60** | **39.14** | **-11.23** | **-2.53** | **-3.62** |

### Example 7. Effect of pH on the performance of EG28+CtCBD cellulase preparation at 50°C and 60°C

Recombinant *Thermoascus aurantiacus* EG28+CBD fusion protein produced in *Trichoderma* (strain RF6377) was tested for its performance in biostoning of denim at different pH values at 50°C and 60°C.

Indigo dyed denim twill (different from the previous examples) that was desized and cut into pieces was used as test material. Cellulase treatments were performed in LP-2 Launder Ometer as follows. About 7.2 g of denim fabric (ca. 12 cm x 12 cm swath), 200 ml of Mc Ilvaine's citrate phosphate buffer, and 90 steel balls (diameter 0.6 cm) were loaded into 1.2 liter containers. The Launder Ometer was run for 120 min at different pH values of 4 to 8 using temperatures 50°C and 60°C. After removing the swathes from the containers they were rinsed with water and soked in water containing NaOH (pH>11) for 10 min with mixing. Thereafter, the swathes were soked in warm water containing liquid detergent (OMO Color) and mixed for 10 min, followed by careful rinsing with warm water for several times. The swathes were dried at room temperature. The biostoning effect was evaluated by measuring the color as reflectance units as described in Example 4. Each measurement value on the face side of denim swath was the average of at least 20 measurements.

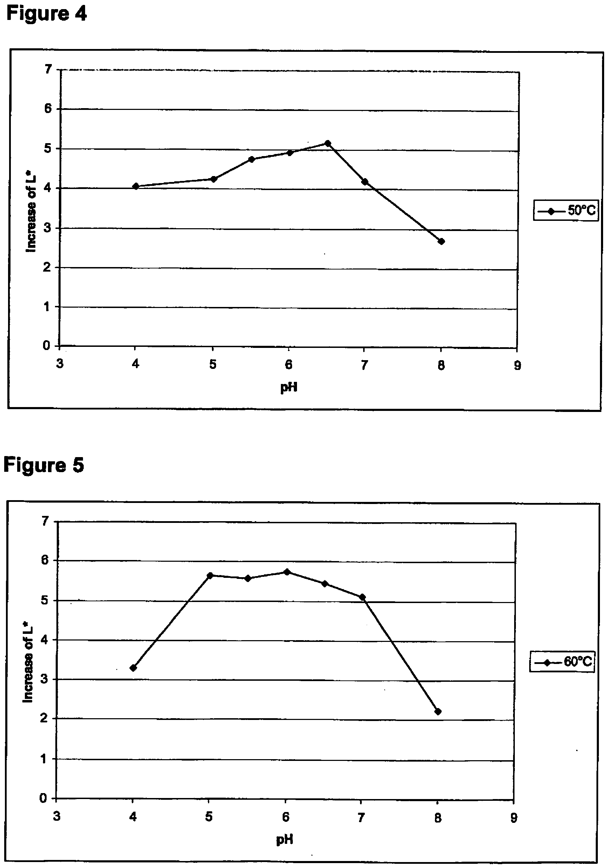

The results shown in Tables 6 and 7 and Figures 4 and 5 show that the most optimal pH range for the EG28+CtCBD cellulase at 50° is 5.5 to 6.5 and at 60°C 5 to 7.

**Table 6. Color measurements on the face side of denim treated with EG28+CtCBD cellulase at different pH values in Launder at 50°C.**

| L* indicates the lightness, -b* is the blue direction. | | | | | | | |
|---|---|---|---|---|---|---|---|
| **Enzyme preparation** | **ECU/g garment** | **Conditions** | **Before cellulase Treatment** | | **After cellulase treatment** | | **Increase of L*** |
| | | | **L*** | **b*** | **L*** | **b*** | |
| Buffer Control | 0 | pH 6 | 17.28 | -13.07 | **18.77** | -14.13 | **1.49** |
| Buffer Control | 0 | pH 8 | 17.01 | -13.4 | **18.18** | -14.45 | **1.17** |
| EG28+CtCBD | 1000 | pH 4 | 16.79 | -13.59 | **20.86** | -16.40 | **4.07** |
| EG28+CtCBD | 1000 | pH 5 | 17.34 | -13.18 | **21.59** | -16.44 | **4.25** |
| EG28+CtCBD | 1000 | pH 5.5 | 17.30 | -13.25 | **22.06** | -16.39 | **4.76** |
| EG28+CtCBD | 1000 | pH6 | 17.35 | -13.42 | **22.29** | -16.47 | **4.94** |
| EG28+CtCBD | 1000 | pH 6.5 | 17.31 | -13.41 | **22.48** | -16.65 | **5.17** |
| EG28+CtCBD | 1000 | pH 7 | 17.34 | -13.25 | **21.55** | -16.23 | **4.21** |
| EG28+CtCBD | 1000 | pH 8 | 16.91 | -13.63 | **19.62** | -15.72 | **2.71** |

**Table 7. Color measurements on the face side of denim treated with EG28+CtCBD cellulase at different pH values in Launder at 60°C.**

| L* indicates the lightness, -b* is the blue direction. | | | | | | | |
|---|---|---|---|---|---|---|---|
| **Enzyme preparation** | **ECU/g garment** | **Conditions** | **Before cellulase** | | **After cellulase** | | **Increase of L*** |
| | | | **L*** | **b*** | **L*** | **b*** | |
| EG28+CtCBD | 1000 | pH 4 | 17.19 | -13.58 | **20.48** | -15.77 | **3.29** |
| EG28+CtCBD | 1000 | pH 5 | 17.10 | -13.24 | **22.74** | -16.59 | **5.64** |
| EG28+CtCBD | 1000 | pH 5.5 | 17.10 | -13.54 | **22.66** | -16.61 | **5.56** |
| EG28+CtCBD | 1000 | pH 6 | 17.10 | -13.65 | **22.83** | -16.74 | **5.73** |
| EG28+CtCBD | 1000 | pH 6.5 | 17.19 | -13.47 | **22.65** | -11.72 | **5.46** |
| EG28+CtCBD | 1000 | pH 7 | 16.99 | -13.72 | **22.10** | -16.66 | **5.11** |
| EG28+CtCBD | 1000 | pH 8 | 17.13 | -13.68 | **19.35** | -15.15 | **2.22** |
| Buffer Control | 0 | pH 6 | 17.36 | -13.6 | **18.95** | -14.28 | **1.59** |

### Example 8. Performance of EG28+CtCBD cellulase preparation in denim finishing at different temperatures

Recombinant *Thermoascus aurantiacus* EG28+CtCBD fusion protein produced in *Trichoderma* (strain RF6377) as described in Example 1 was tested for its ability to create an abraded look similar to that provided by pumice stones in biostoning of denim at different temperatures. Efficient denim finishing enzymes, commercial EGII enriched acid cellulase preparation (US. 5,874,293) and neutral cellulase preparation ECOSTONE^{®} C1 were used as reference.

The test system for biostoning was as in Example 4, except that different Denim twill and a washing time of 55 min were used. The enzymatic activity (endoglucanase unit, ECU) of the EG28+CtCBD and EGII enriched enzyme preparations was measured as in Example 4. The activity (neutral cellulase unit, NCU) of the neutral cellulase preparation ECOSTONE^{®} C1 was measured as the release of reducing sugars from carboxymethyl cellulose as previously described (Bailey and Nevalainen, 1981; Haakana et al. 2004). The ECOSTONE^{®} C1, EG28+CtCBD, or EGII enriched preparations were dosed at 250 NCU/g, 500 ECU/g, or 1000 ECU/g fabric, respectively. The effect of the cellulase treatment was evaluated as in Example 4.

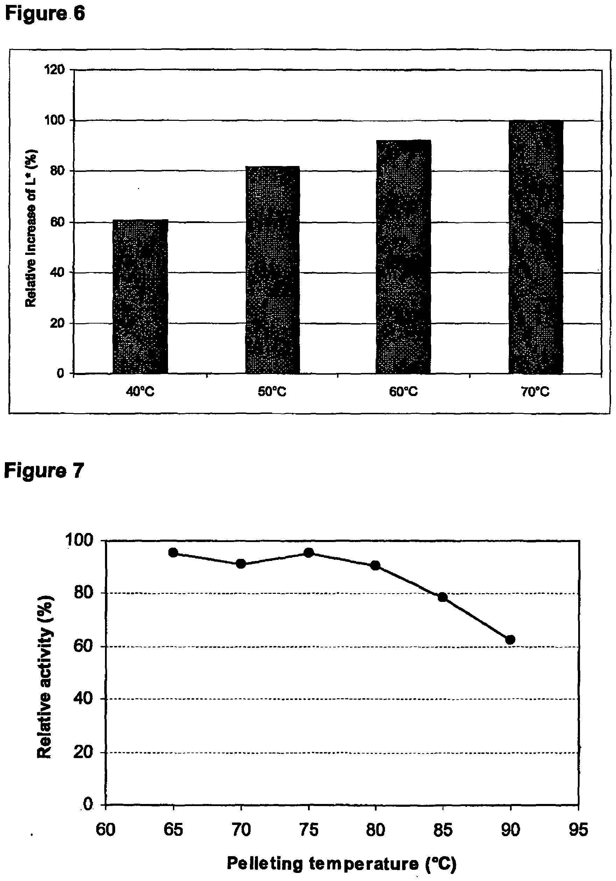

The performance of EG28+CtCBD at temperatures of 40 to 70°C, pH 6 is shown in Table 8 and Figure 6. The preferred temperature range for the enzyme is 50 to 70°C. Lower temperatures like 40°C can be used to obtain an abrasion effect with less colorpull if a darker look is desirable.

The results are also in agreement with those obtained in Example 6, where the performance of EG28+CtCBD in biostoning was shown to be about two times more effective than that of the commercial EGII enriched acid cellulase preparation. Acid cellulases generally have a tendency of backstaining. Neutral cellulases, like ECOSTONE^{®} C1, typically cause low backstaining therefore resulting in good contrast. The results obtained here show that a similar effect was obtained with the EG28+CtCBD cellulase as with the neutral cellulases (Table 8).

**Table 8. Color measurements of denim treated with EG28+CtCBD cellulase at different temperatures.**

| Treatment with EGII enriched acid cellulase and ECOSTONE^{®} C1 neutral cellulase preparation was used as reference. L* indicates the lightness, -b* is the blue direction, +b* is the yellow direction. | | | | | | | | |
|---|---|---|---|---|---|---|---|---|
| **Enzyme preparation** | **Activity/g garment** | **Conditions** | **Before cellulase Treatment** | | **After cellulase Treatment** | | **deltaL*** | **deltab*** |
| | | | **L*** | **b*** | **L*** | **b*** | | |
| **Face side:** | | | | | | | | |
| EG28+CBD | 500 ECU/g | 70C°, pH 6.2 | 17.38 | -13.49 | 27.42 | -17.27 | **10.05** | -3.78 |
| EG28+CBD | 500 ECU/g | 60C°, pH 6.2 | 16.76 | -13.31 | **26.01** | -17.49 | **9.25** | -4.18 |
| EG28+CBD | 500 ECU/g | 50C°, pH 6.2 | 16.78 | -13.40 | 25.00 | -17.44 | **8.22** | -4.04 |
| EG28+CBD | 500 ECU/g | 40C°, pH 6.2 | 16.76 | -13.21 | 22.86 | -17.25 | **6.11** | -4.04 |
| EGII enriched | **1000 ECU/g** | 60C°, pH 5.1 | 17.24 | -13.38 | **26.59** | -17.64 | **9.35** | -4.26 |
| ECOSTONE C1 | **250 NCU/g** | 60C°, pH 6.5 | 17.38 | -13.53 | **26.91** | -16.98 | **9.53** | -3.45 |
| **Reverse side:** | | | | | | | | |
| EG28+CBD | 500 ECU/g | 70C°, pH 6.2 | 45.67 | -6.33 | 41.78 | -13.83 | -3.90 | -7.51 |
| EG28+CBD | 500 ECU/g | 60C°, pH 6.2 | 45.60 | -5.95 | **42.72** | -13.05 | **-2.88** | -7.10 |
| EG28+CBD | 500 ECU/g | 50C°, pH 6.2 | 45.67 | -5.92 | 41.81 | -13.35 | -3.86 | -7.44 |
| EG28+CBD | 500 ECU/g | 40C°, pH 6.2 | 45.80 | -5.72 | 41.66 | -12.52 | -4.14 | -6.81 |
| EGII enriched | **1000 ECU/g** | 60C°, pH 5.1 | 46.10 | -6.19 | **39.89** | -15.24 | **-6.21** | -9.06 |
| ECOSTONE C1 | **250 NCU/g** | 60C°, pH 6.5 | 45.63 | -6.46 | **42.49** | -12.76 | **-3.14** | -6.31 |

### Example 9. Performance of EG28 and EG28+CtCBD cellulases in biofinishing (depilling)

The performance of EG28 cellulase from strain RF6188 and EG28+CtCBD preparation from strain RF6377 in depilling of cotton knitwear was tested. The cellulase treatments were performed with Electrolux's Wascator FOM 71 CLS washer extractor under conditions described in Table 9.

Pieces of two kinds of low quality Polo-neck sweaters with fuzzy surface, made of 100% cotton jersey-based fabric or rib made of 95% cotton and 5% of lycra were used as test material with filling material. Samples were first prewashed for 10 min at 60°C with 1 ml/l surfactants/wetting agents (Sandoclean PCJ from Sandos and Imacol CN from Clariant) and rinsed three times with water. After that the cotton knits were treated with cellulase at 60°C for 60 minutes, in the presence of the same textile auxiliaries as used in prewash. The enzyme was inactivated as described in Example 4, except for the temperature, which was 60°C during the alkaline rinse. The pieces of knitwear were rinsed three times and thereafter dried in the dumbler.

**Table 9. Test conditions/process parameters used in biofinishing treatments.**

| **Process parameter** | |
|---|---|
| Fabric load | 1.0 kg |
| Water | 15 liter |
| Sandoclean PCJ and Imacol CN | 1 ml/l |
| Buffer/pH control (pH 5 -5.3) | ca. 3 ml Acetic acid (80 %) |
| Time | 60 min |
| Temperature | 60°C |
| Cellulase dosage | 1200 ECU/g fabric |

The effect of the cellulase treatment was evaluated visually with the naked eye and with a loupe. A prewashed sample without enzyme was used as control.

The results showed that the EG28+CtCBD preparation improved depilling properties. The surface fuzzing of the knitwear was considerably reduced after treatment with the EG28+CtCBD preparation compared to the control (treatment without enzyme). Also the EG28 preparation had a depilling effect.

### Example 10. Feed pelleting stability test

Two cellulose preparations, EG28 (strain RF6188) and EG28+CtCBD (strain RF6377) were separately tested in an experiment, which simulates an industrial feed production process. Prior to testing the spray-dried EG28 or EG28+CtCBD preparations were ground with wheat flour in order to improve homogeneity at feed level. EG28 or EG28+CtCBD enzymes premixed with flour were added at a dosage of 200 g/t and at 500 g/t of feed, respectively. Enzyme overdosing was used to facilitate analysis from samples pelleted at high temperatures, where activity may have been substantially reduced.

The mill and mixer are used for producing meal mixtures as part of a semi-industrial feed plant with a nominal pelleting capacity of 5 t/h. The mill is a Champion hammer mill equipped with Ø 3.0 mm die. The ground raw material is mixed in a 2,500 litre horizontal mixer at 27 rpm before being transferred to the mini feed milling plant. The plant comprises a horizontal mixer (volume 700 L, speed 48 rpm, mixing batches of 300 kg), a Skjold TR dozing screw and a Kahl cascade mixer (length 130 cm, diameter 30 cm, speed 155 rpm, equipped with 37 adjustable pallets). The dwell time for 300 kg/h is approx. 30 sec. Mounted on the side of the cascade mixer is a manifold with a water discharger and 3 steam valves from which steam is lead to the meal. Steam is provided by a high-pressure boiler with a maximum capacity of 400 kg steam/h. Tests are conducted with 2 atmospheres overpressure and the steam is led via a pressure reduction valve, which controls the addition of steam to the cascade mixer. Three valves on the manifold are used for fine adjustment of the desired meal temperature. Meal temperature is measured by a digital thermometer placed in the outlet of the cascade mixer, just before the meal entered to pellet die. The pellet press is a Simon Heesen, type labor (monoroll) with a Ø 3 mm * 35 mm die and a 7.5 kW motor. Internal diameter of die: 173 mm, height of press roll: 50 mm, diameter of press roll: 140 mm, run rate 500 rpm and nominal capacity: 300 kg/h. Samples are taken after the pellet press and cooled in a partitioned cooling box with perforated bottom, ventilator: 1500 m³ air/h.

A 300 kg batch of a wheat-based mash feed was produced. A premix was produced from 10 kg of this meal and 200 g (or 500 g) of the test enzyme in a 70 litre compulsory mixer. Mixer speed: 45 rpm. Mixing time: 10 min. This premix was added with 290 kg of the feed into the horizontal mixer in the mini feed milling plant and mixed for 10 minutes. After sampling of the mash, the feed was pelleted through the pellet press (die 0 3 mm). The meal was heated to the target temperature (65 to 90°C) by adjusting steam addition to the cascade mixer. For each temperature, a sample was first taken 10 minutes after the target pelleting temperature (as measured in the meal just prior to pelleting) was achieved. Samples were taken during 1.0 min. corresponding to 5.0 kg of pelleted feed. The sample was taken as sub-samples of approx. 500 grams and placed in a cooling box for 10-15 seconds after the pellets had left the pellet press. All samples were aerated and cooled at ambient temperature for 15 minutes. The samples were homogeneously sub-sampled on a riffler sample divider and filled into plastic bags.

The samples were analysed as follows: 2.5 g of well ground sample and 20 ml of acetate buffer (0.05 M, pH 5.0) buffer were stirred for 30 min at room temperature, centrifuged (10 min, 4000 rpm) and diluted. 1.0 ml of cleared sample in triplicate was equilibrated for 5 min in a water bath at 40°C. The reaction was initiated by addition of a Beta-Glucazyme tablet (Megazyme, Ireland) without stirring. After exactly 30 min the reaction was stopped by adding 5.0 ml of Trizma Base 1% (w/v) (Sigma-Aldrich) with vigorous stirring on a vortex mixer. The tube was left at room temperature for about 5 min; the slurry was stirred again and filtered through Whatman 1 filter paper. The absorbance of the filtrate was measured at 590 nm. The results were analyzed using a standard curve.

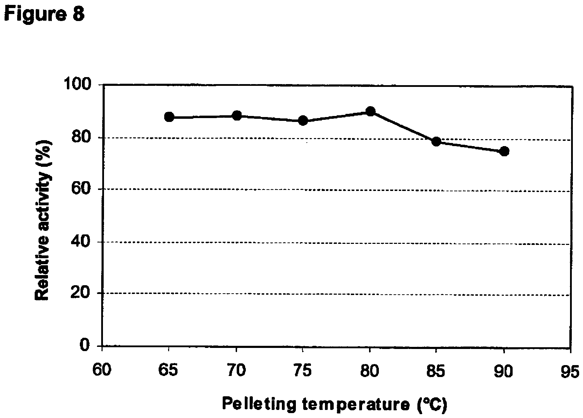

Enzyme activity recoveries before and after pelleting are presented in Table 10. The influence of pelleting temperature on the recovery of the beta-glucanase activity has also been presented in Figures 7 and 8. The EG28 and EG28+CtCBD cellulases were stable up to 80°C. The EG28+CtCBD cellulase tended to be more stable than EG28 at the pelleting temperature of 90°C.

**Table 10. Enzyme activity recoveries before (meal from horizontal mixer) and after pelleting at temperatures ranging from 65 to 90°C.**

| Pelleting temperature | Beta-Glucanase activity (FBU/kg of feed) | |
|---|---|---|
| | (EG28) | (EG28+CtCBD) |
| Meal from horizontal mixer | 239 000 ± 9 000 (100%) | 1 148 000 ± 48 000 (100%) |
| Pellet (65°C) | 227 000 ± 16 000 (95%) | 1 007 000 ± 67 000 (88%) |
| Pellet (70°C) | 217 000 ± 5 000 (91%) | 1 012 000 ± 54 000 (88%) |
| Pellet (75°C) | 227 000 ± 14 000 (95%) | 993 000 ± 49 000 (86%) |
| Pellet (80°C) | 215 000 ± 6 000 (90%) | 1 030 000 ± 87 000 (90%) |
| Pellet (85°C) | 188 000 ± 7 000 (79%) | 904 000 ± 56 000 (79%) |
| Pellet (90°C) | 149 000 ± 8 200 (62%) | 861 000 ± 32 000 (75%) |

**List of deposited organisms**

| **Strain** | **Plasmid contained** | **Deposition authority** | **Deposition date** | **Deposition number** |
|---|---|---|---|---|
| *Acremonium thermophilum* ALKO4245 | - | CBS⁽¹⁾ | Sep 20, 2004 | CBS 116240 |
| *Thermoascus aurantiacus* ALKO4242 | - | CBS⁽¹⁾ | Sep 20, 2004 | CBS 116239 |
| *Chaetomium thermophilum* ALKO4265 | - | CBS⁽²⁾ | Nov 8, 1995 | CBS 730.95 |
| *Escherichia coli* | pALK1946 | DSMZ⁽³⁾ | Apr 7, 2006 | DSM 18159 |
| *Escherichia coli* | pALK1926 | DSMZ | May 13, 2005 | DSM 17326 |

| | | | | |
|---|---|---|---|---|
| ⁽¹⁾the Centralbureau Voor Schimmelcultures at Uppsalalaan 8, 3584 CT, Utrecht, the Netherlands ⁽²⁾the Centralbureau Voor Schimmelcultures at Oosterstraat 1, 3742 SK BAARN, The Netherlands ⁽³⁾Deutsche Sammlung von Mikroorganismen und Zellkulturen GmbH (DSMZ), Mascheroder Weg 1 b, D-38124 Braunschweig, Germany | | | | |

### References

Altschul SF, W Gish, W Miller, EW Myers and DJ Lipman. 1990. Basic local alignment search tool. J. Mol. Biol. 215:403-410.
Bailey MJ and KMH Nevalainen 1981. Induction, isolation and testing of stable Trichoderma reesei mutants with improved production of solubilizing cellulase. Enz Microbiol Technol. 3: 153-157.
Bendtsen J.D., Nielsen H., von Heijne G. and Brunak S. (2004) Improved prediction of signal peptides: SignalP 3.0. J. Mol. Biol. 340: 783-795.
Gasteiger, E, A Gattiker, C Hoogland, I Ivanyi, RD Appel and A Bairoch. 2003. ExPASy: the proteomics server for in-depth protein knowledge and analysis. Nucleic Acids Res. 31:3784-3788.
Haakana H, A Miettinen-Oinonen, V Joutsjoki, A Mäntylä, P Suominen, and J Vehmaanperä. 2004. Cloning of cellulase genes from Melanocarpus albomyces and their efficient expression in Trichoderma reesei. Enz Microbiol Technol. 34: 159-167.
Henrissat B. (1991) A classification of glycosyl hydrolases based on amino acid sequence similarities. Biochem. J. 280: 309-316.
Henrissat B. and Bairoch A. (1993) New families in the classification of glycosyl hydrolases based on amino acid sequence similarities. Biochem. J. 293: 781-788.
Henrissat B. and Bairoch A. (1996).Updating the sequence-based classification of glycosyl hydrolases. Biochem. J. 316: 695-696.
Hong J., H. Tamaki, K. Yamamoto, and H. Kumagai. 2003. Cloning of a gene encoding a thermo-stabile endo-β-1,4-glucanase from Thermoascus aurantiacus and its expression in yeast. Biotech. Letters 25:657-661.
IUPAC (International Union of Pure and Applied Chemistry) (1987). Measurement of cellulase activities. Pure and Appl. Chem. 59:257-268.
Joutsjoki, W, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Malardier L, Daboussi MJ , Julien J, Roussel F, Scazzocchio C and Brygoo Y. 1989. Cloning of the nitrate reductase gene (niaD) of Aspergillus nidulans and its use for transformation of Fusarium oxysporum. Gene 15:147-156.
Needleman S. and Wunsch C. (1970) A general method applicable to the search for similarities in the amino acid sequence of two proteins. Journal of Molecular Biology 48, 443-453.
Nielsen H., Engelbrecht J., Brunak S. and von Heijne G. (1997) Identification of prokaryotic and eykaryotic signal peptides and prediction of thier cleavage sites. Protein Engineering 10: 1-6.
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Raeder U and P Broda. 1985. Rapid preparation of DNA from filamentous fungi. Lett. Appl. Microbiol. 1:17-20.
Rice P, Longden I and Bleasby A. (2000). EMBOSS: The European Molecular Biology Open Software Suite. Trends in Genetics 16:276-277.
Sambrook J, EF Fritsch and T Maniatis. 1989. Molecular cloning, a laboratory manual. Cold Spring Harbor Laboratory, New York, US.
Sambrook J and DW Russell. 2001. Molecular cloning, a laboratory manual. Cold Spring Harbor Laboratory, New York, US.
Srisodsuk M, Reinikainen T, Penttilä M and Teeri T. (1993) Role of the interdomain linker peptide of Trichoderma reesei cellobiohydrolase I in its interaction with crystalline cellulose. J. Biol. Chem. Oct 5;268(28): 20756-61.
Stålbrand H, Siika-aho M, Tenkanen M and L Viikari. 1993. J. Bio-technol. 29:229-242.
Ward M, Shan W, Dauberman J, Weiss G, Larenas E, Bower B, Rey M, Clarkson K and Bott R. (1993) Cloning, sequence and preliminary structural analysis of a small, high pl endoglucanase (EGIII) from Trichoderma reesei. Proceedings of the second TRICEL symposium on TRICHODERMA REESEI CELLULASES AND OTHER HYDROLASES, Espoo, Finland, 1993, ed. by P. Suominen and T. Reinikainen. Foundation for Biotechnical and Industrial Fermentation Research 8 (1993): 153-158.

## Claims

1. A cellulase fusion protein comprising a first amino acid sequence of an endoglucanase core having at least 75% identity to SEQ ID NO: 2, or a fragment thereof having cellulase activity, and a second amino acid sequence comprising a linker and cellulose binding domain (CBD) having at least 75% identity to SEQ ID NO: 15, or a fragment thereof having cellulose binding activity.

2. The fusion protein of claim 1, wherein the endoglucanase core has at least 80% identity to SEQ ID NO: 2, or a fragment thereof having cellulase activity.

3. The fusion protein of claim 1, wherein the linker and CBD have at least 80% identity to SEQ ID NO: 15, or a fragment thereof having cellulose binding activity.

4. The fusion protein of claim 1, wherein the endoglucanase core comprises the sequence of SEQ ID NO: 2, and the linker and CBD comprise the sequence of SEQ ID NO: 15.

5. The fusion protein of claim 1 comprising the amino acid sequence of SEQ ID NO: 4, or a fragment thereof having cellulase and cellulose binding activity.

6. The fusion protein of claim 1, wherein the endoglucanase core is encoded by a gene similar to that included in *E. coli* DSM 17326, or which fusion protein is encoded by a fusion gene similar to that included in *E. coli* DSM 18159.

7. An isolated polynucleotide selected from the group consisting of:
a) a nucleotide sequence of SEQ ID NO: 3, or a sequence encoding a cellulase fusion protein of claim 1,
b) a complementary strand of a).

8. An expression vector comprising a nucleotide sequence of claim 7.

9. A host cell comprising the expression vector of claim 8.

10. A method of producing a fusion protein of any one of claims 1 - 6, comprising the steps of transforming a host cell with an expression vector encoding said fusion protein and culturing said host cell under conditions enabling expression of said fusion protein, and optionally recovering and purifying the fusion protein produced.

11. An enzyme preparation comprising the fusion protein of any one of claims 1 - 6.

12. A process for treating cellulosic material, wherein said process comprises contacting the cellulosic material with the fusion protein of any one of claims 1 - 6, or the enzyme preparation of claim 11.

13. The process of claim 12, wherein the cellulosic material is textile material, plants used in animal feed, or wood-derived pulp or secondary fiber.

14. Animal feed comprising the fusion protein of any one of claims 1 to 6.

15. An *Eschrichia coli* strain having accession number DSM 18159.

## Patentansprüche

1. Cellulase-Fusionsprotein, umfassend eine erste Aminosäuresequenz eines Endoglucanase-Kerns mit wenigstens 75% Identität mit SEQ ID Nr. 2 oder ein Fragment davon, das Cellulase-Aktivität aufweist, und eine zweite Aminosäuresequenz, die einen Linker und eine Cellulosebindungsdomäne (CBD) mit wenigstens 75% Identität mit SEQ ID Nr. 15 oder ein Fragment davon, das Cellulosebindungsaktivität aufweist, umfasst.

2. Fusionsprotein gemäß Anspruch 1, wobei der Endoglucanase-Kern wenigstens 80% Identität mit SEQ ID Nr. 2 aufweist, oder ein Fragment davon, das Cellulase-Aktivität aufweist.

3. Fusionsprotein gemäß Anspruch 1, wobei der Linker und CBD wenigstens 80% Identität mit SEQ ID Nr. 15 aufweist, oder ein Fragment davon, das Cellulosebindungsaktivität aufweist.

4. Fusionsprotein gemäß Anspruch 1, wobei der Endoglucanase-Kern die Sequenz von SEQ ID Nr. 2 umfasst und der Linker und CBD die Sequenz von SEQ ID Nr. 15 umfassen.

5. Fusionsprotein gemäß Anspruch 1, das die Aminosäuresequenz von SEQ ID Nr. 4 oder ein Fragment davon, das Cellulase-Aktivität und Cellulosebindende Aktivität aufweist, umfasst.

6. Fusionsprotein gemäß Anspruch 1, wobei der Endoglucanase-Kern von einem ähnlichen Gen codiert wird wie das, das in *E. coli* DSM 17326 enthalten ist, oder dessen Fusionsprotein von einem ähnlichen Fusions-Gen codiert wird wie das, das in *E. coli* DSM 18159 enthalten ist.

7. Isoliertes Polynucleotid, das aus der Gruppe ausgewählt ist, die aus
a) einer Nucleotidsequenz von SEQ ID Nr. 3 oder einer Sequenz, die ein Cellulase-Fusionsprotein gemäß Anspruch 1 codiert;
b) einem zu a) komplementären Strang
besteht.

8. Expressionsvektor, der eine Nucleotidsequenz gemäß Anspruch 7 umfasst.

9. Wirtszelle, die den Expressionsvektor gemäß Anspruch 8 umfasst.

10. Verfahren zur Herstellung eines Fusionsproteins gemäß einem der Ansprüche 1 bis 6, umfassend die Schritte des Transformierens einer Wirtszelle mit einem Expressionsvektor, der das Fusionsprotein codiert, und des Kultivierens der Wirtszelle unter Bedingungen, die die Expression des Fusionsproteins ermöglichen, und gegebenenfalls das Gewinnen und Reinigen des erzeugten Fusionsproteins.

11. Enzympräparat, das das Fusionsprotein gemäß einem der Ansprüche 1 bis 6 umfasst.

12. Verfahren zur Behandlung von Cellulosematerial, wobei das Verfahren das In-Kontakt-Bringen des Cellulosematerials mit dem Fusionsprotein gemäß einem der Ansprüche 1 bis 6 oder mit dem Enzympräparat gemäß Anspruch 11 umfasst.

13. Verfahren gemäß Anspruch 12, wobei es sich bei dem Cellulosematerial um Textilmaterial, in Tierfutter verwendete Pflanzen oder Zellstoff oder eine sekundäre Faser handelt.

14. Tierfutter, das das Fusionsprotein gemäß einem der Ansprüche 1 bis 6 umfasst.

15. *Escherichia-coli*-Stamm mit der Akzessionsnummer DSM 18159.

## Revendications

1. Protéine de fusion de cellulase comprenant une première séquence d'aminoacides d'un noyau endoglucanase ayant une identité d'au moins 75 % avec la SEQ ID N° 2, ou un de ses fragments ayant une activité de cellulase, et une seconde séquence d'aminoacides comprenant un segment de liaison et un domaine de liaison à la cellulose (CBD) présentant une identité d'au moins 75 % avec la SEQ ID N° 15, ou un de ses fragments ayant une activité de liaison à la cellulose.

2. Protéine de fusion suivant la revendication 1, dans laquelle le noyau endoglucanase a une identité d'au moins 80 % avec la SEQ ID N° 2, ou un de ses fragments ayant une activité de cellulase.

3. Protéine de fusion suivant la revendication 1, dans laquelle le segment de liaison et le CBD ont une identité d'au moins 80 % avec la SEQ ID N° 15, ou un de ses fragments ayant une activité de liaison à la cellulose.

4. Protéine de fusion suivant la revendication 1, dans laquelle le noyau endoglucanase comprend la séquence de la SEQ ID N° 2, et le segment de liaison et le CBD comprennent la séquence de la SEQ ID N° 15.

5. Protéine de fusion suivant la revendication 1, comprenant la séquence d'aminoacides de la SEQ ID N° 4, ou un de ses fragments ayant une activité de cellulase et de liaison à la cellulose.

6. Protéine de fusion suivant la revendication 1, dans laquelle le noyau endoglucanase est codé par un gène similaire à celui inclus dans *E. coli* DSM 17326, ou bien protéine de fusion qui est codée par un gène de fusion similaire à celui inclus dans *E. coli* DSM 18159.

7. Polynucléotide isolé choisi dans le groupe consistant en :
a) une séquence de nucléotides de la SEQ ID N° 3, ou une séquence codant pour une protéine de fusion de cellulase de la revendication 1,
b) un brin complémentaire de a).

8. Vecteur d'expression comprenant une séquence de nucléotides de la revendication 7.

9. Cellule hôte comprenant le vecteur d'expression de la revendication 8.

10. Procédé pour la production d'une protéine de fusion de l'une quelconque des revendications 1 à 6, comprenant les étapes de transformation d'une cellule hôte avec un vecteur d'expression codant pour ladite protéine de fusion et la culture de ladite cellule hôte dans des conditions permettant l'expression de ladite protéine de fusion et, facultativement, la récupération et la purification de la protéine de fusion produite.

11. Préparation d'enzyme comprenant la protéine de fusion de l'une quelconque des revendications 1 à 6.

12. Procédé pour traiter une matière cellulosique, ledit procédé comprenant la mise en contact de la matière cellulosique avec la protéine de fusion de l'une quelconque des revendications 1 à 6, ou la préparation d'enzyme de la revendication 11.

13. Procédé suivant la revendication 12, dans lequel la matière cellulosique est une matière textile, des plantes utilisées dans la nourriture pour animaux ou de la pâte ou fibre secondaire dérivée du bois.

14. Nourriture pour animaux comprenant la protéine de fusion de l'une quelconque des revendications 1 à 6.

15. Souche de *Escherichia coli* ayant le numéro de dépôt DSM 18159.


### SEQUENCE LISTING

<110> AB Enzymes Oy
<120> Enzyme fusion proteins and their use
<130> 2060570
<160> 15
<170> Patentin version 3.2
<210> 1
   <211> 1317
   <212> DNA
   <213> Thermoascus aurantiacus
<220>
   <221> gene
   <222> (1)..(1317)
<220>
   <221> Intron
   <222> (107)..(161)
<220>
   <221> Intron
   <222> (221)..(280)
<220>
   <221> Intron
   <222> (434)..(492)
<220>
   <221> Intron
   <222> (558)..(631)
<220>
   <221> Intron
   <222> (730)..(790)
<400> 1
   atgaagctcg gctctctcgt gctcgctctc agcgcagcta ggcttacact gtcggcccct      60
   ctcgcagaca ggaagcagga gaccaagcgt gcgaaagtat tccaatgttc gtaacatcca     120
   cgtctggctt gctggcttac tggcaactga caatggcgaa gggttcggtt caaacgagtc     180
   cggtgctgaa ttcggaagcc agaaccttcc aggagtcgag gtcagcatgc ctgtactctc     240
   tgcattatat taatatctca agaggcttac tctttcgcag ggaaaggatt atatatggcc     300
   tgatcccaac accattgaca cattgatcag caaggggatg aacatctttc gtgtcccctt     360
   tatgatggag agattggttc ccaactcaat gaccggctct ccggatccga actacctggc     420
   agatctcata gcggtacatt tcaattccac catgtttgga gctgtcttcg ttgtgctgac     480
   atttaatggt agactgtaaa tgcaatcacc cagaaaggtg cctacgccgt cgtcgatcct     540
   cataactacg gcagatagtg aggtccccgg ttctggtatt gctgctgtat atctaagtag     600
   atatgtgttt ctaacatttc cacgatttca gctacaattc tataatctcg agcccttccg     660
   atttccagac cttctggaaa acggtcgcct cacagtttgc ttcgaatcca ctggtcatct     720
   tcgacactag taagctgaac acccgaaatt aactgagtct gagcatgtct gacaagacga     780
   tccatgaaag ataacgaata ccacgatatg gaccagacct tagtcctcaa tctcaaccag     840
   gccgctatcg acggcatccg ttccgccgga gccacttccc agtacatctt tgtcgagggc     900
   aattcgtgga ccggggcatg gacctggacg aacgtgaacg ataacatgaa aagcctgacc     960
   gacccatctg acaagatcat atacgagatg caccagtacc tggactctga cggatccggg    1020
   acatcagcga cctgcgtatc ttcgaccatc ggtcaagagc gaatcaccag cgcaacgcaa    1080
   tggctcaggg ccaacgggaa gaagggcatc atcggcgagt ttgcgggcgg agccaacgac    1140
   gtctgcgaga cggccatcac gggcatgctg gactacatgg cccagaacac ggacgtctgg    1200
   actggcgcca tctggtgggc ggccgggccg tggtggggag actacatatt ctccatggag    1260
   ccggacaatg gcatcgcgta tcagcagata cttcctattt tgactccgta tctttga       1317
<210> 2
   <211> 335
   <212> PRT
   <213> Thermoascus aurantiacus
<400> 2
   Met Lys Leu Gly Ser Leu Val Leu Ala Leu Ser Ala Ala Arg Leu Thr
   1               5                   10                  15
   Leu Ser Ala Pro Leu Ala Asp Arg Lys Gln Glu Thr Lys Arg Ala Lys
               20                  25                  30
   Val Phe Gln Trp Phe Gly Ser Asn Glu Ser Gly Ala Glu Phe Gly Ser
           35                  40                  45
   Gln Asn Leu Pro Gly Val Glu Gly Lys Asp Tyr Ile Trp Pro Asp Pro
       50                  55                  60
   Asn Thr Ile Asp Thr Leu Ile Ser Lys Gly Met Asn Ile Phe Arg Val
   65                  70                  75                  80
   Pro Phe Met Met Glu Arg Leu Val Pro Asn Ser Met Thr Gly Ser Pro
                   85                  90                  95
   Asp Pro Asn Tyr Leu Ala Asp Leu Ile Ala Thr Val Asn Ala Ile Thr
               100                 105                 110
   Gln Lys Gly Ala Tyr Ala Val Val Asp Pro His Asn Tyr Gly Arg Tyr
           115                 120                 125
   Tyr Asn Ser Ile Ile Ser Ser Pro Ser Asp Phe Gln Thr Phe Trp Lys
       130                 135                 140
   Thr Val Ala Ser Gln Phe Ala Ser Asn Pro Leu Val Ile Phe Asp Thr
   145                 150                 155                 160
   Asn Asn Glu Tyr His Asp Met Asp Gln Thr Leu Val Leu Asn Leu Asn
                   165                 170                 175
   Gln Ala Ala Ile Asp Gly Ile Arg Ser Ala Gly Ala Thr Ser Gln Tyr
   180 185 190
   Ile Phe Val Glu Gly Asn Ser Trp Thr Gly Ala Trp Thr Trp Thr Asn
   195 200 205
   Val Asn Asp Asn Met Lys Ser Leu Thr Asp Pro Ser Asp Lys Ile Ile
   210 215 220
   Tyr Glu Met His Gln Tyr Leu Asp Ser Asp Gly Ser Gly Thr Ser Ala
   225 230 235 240
   Thr Cys Val Ser Ser Thr Ile Gly Gln Glu Arg Ile Thr Ser Ala Thr
   245 250 255
   Gln Trp Leu Arg Ala Asn Gly Lys Lys Gly Ile Ile Gly Glu Phe Ala
   260 265 270
   Gly Gly Ala Asn Asp Val Cys Glu Thr Ala Ile Thr Gly Met Leu Asp
   275 280 285
   Tyr Met Ala Gln Asn Thr Asp Val Trp Thr Gly Ala Ile Trp Trp Ala
   290 295 300
   Ala Gly Pro Trp Trp Gly Asp Tyr Ile Phe Ser Met Glu Pro Asp Asn
   305 310 315 320
   Gly Ile Ala Tyr Gln Gln Ile Leu Pro Ile Leu Thr Pro Tyr Leu
   325 330 335
<210> 3
   <211> 1621
   <212> DNA
   <213> Thermoascus aurantiacus
<220>
   <221> gene
   <222> (1)..(1621)
<220>
   <221> Intron
   <222> (107)..(161)
<220>
   <221> Intron
   <222> (221)..(280)
<220>
   <221> Intron
   <222> (434)..(492)
<220>
   <221> Intron
   <222> (558)..(631)
   <220>
   <221> Intron
   <222> (730)..(790)
<220>
   <221> intro
   <222> (1549)..(1612)
<400> 3
   atgaagctcg gctctctcgt gctcgctctc agcgcagcta ggcttacact gtcggcccct     60
   ctcgcagaca ggaagcagga gaccaagcgt gcgaaagtat tccaatgttc gtaacatcca    120
   cgtctggctt gctggcttac tggcaactga caatggcgaa gggttcggtt caaacgagtc    180
   cggtgctgaa ttcggaagcc agaaccttcc aggagtcgag gtcagcatgc ctgtactctc    240
   tgcattatat taatatctca agaggcttac tctttcgcag ggaaaggatt atatatggcc    300
   tgatcccaac accattgaca cattgatcag caaggggatg aacatctttc gtgtcccctt    360
   tatgatggag agattggttc ccaactcaat gaccggctct ccggatccga actacctggc    420
   agatctcata gcggtacatt tcaattccac catgtttgga gctgtcttcg ttgtgctgac    480
   atttaatggt agactgtaaa tgcaatcacc cagaaaggtg cctacgccgt cgtcgatcct    540
   cataactacg gcagatagtg aggtccccgg ttctggtatt gctgctgtat atctaagtag    600
   atatgtgttt ctaacatttc cacgatttca gctacaattc tataatctcg agcccttccg    660
   atttccagac cttctggaaa acggtcgcct cacagtttgc ttcgaatcca ctggtcatct    720
   tcgacactag taagctgaac acccgaaatt aactgagtct gagcatgtct gacaagacga    780
   tccatgaaag ataacgaata ccacgatatg gaccagacct tagtcctcaa tctcaaccag    840
   gccgctatcg acggcatccg ttccgccgga gccacttccc agtacatctt tgtcgagggc    900
   aattcgtgga ccggggcatg gacctggacg aacgtgaacg ataacatgaa aagcctgacc    960
   gacccatctg acaagatcat atacgagatg caccagtacc tggactctga cggatccggg   1020
   acatcagcga cctgcgtatc ttcgaccatc ggtcaagagc gaatcaccag cgcaacgcaa   1080
   tggctcaggg ccaacgggaa gaagggcatc atcggcgagt ttgcgggcgg agccaacgac   1140
   gtctgcgaga cggccatcac gggcatgctg gactacatgg cccagaacac ggacgtctgg   1200
   actggcgcca tctggtgggc ggccgggccg tggtggggag actacatatt ctccatggag   1260
   ccggacaatg gcatcgcgta tcagcagata cttcctattt tgactccgta cgtacctggc   1320
   cttgacggca gcaaccccgg caacccgacc accaccgtcg ttcctcccgc ttctacctcc   1380
   acctcccgtc cgaccagcag cactagctct cccgtttcga ccccgactgg ccagcccggc   1440
   ggctgcacca cccagaagtg gggccagtgc ggcggtatcg gctacaccgg ctgcactaac    1500
   tgcgttgctg gcaccacctg cactcagctc aacccctggt acagccaggt atgtttctct   1560
   tcccccttct agactcgctt ggatttgaca gttgctaaca tctgctcaac agtgcctgta    1620
   a                                                                   1621
<210> 4
   <211> 415
   <212> PRT
   <213> Thermoascus aurantiacus
<400> 4
   Met Lys Leu Gly Ser Leu Val Leu Ala Leu Ser Ala Ala Arg Leu Thr
   1 5 10 15
   Leu Ser Ala Pro Leu Ala Asp Arg Lys Gln Glu Thr Lys Arg Ala Lys
   20 25 30
   Val Phe Gln Trp Phe Gly Ser Asn Glu Ser Gly Ala Glu Phe Gly Ser
   35 40 45
   Gln Asn Leu Pro Gly Val Glu Gly Lys Asp Tyr Ile Trp Pro Asp Pro
   50 55 60
   Asn Thr Ile Asp Thr Leu Ile Ser Lys Gly Met Asn Ile Phe Arg Val
   65 70 75 80
   Pro Phe Met Met Glu Arg Leu Val Pro Asn Ser Met Thr Gly Ser Pro
   85 90 95
   Asp Pro Asn Tyr Leu Ala Asp Leu Ile Ala Thr Val Asn Ala Ile Thr
   100 105 110
   Gln Lys Gly Ala Tyr Ala Val Val Asp Pro His Asn Tyr Gly Arg Tyr
   115 120 125
   Tyr Asn Ser Ile Ile Ser Ser Pro Ser Asp Phe Gln Thr Phe Trp Lys
   130 135 140
   Thr Val Ala Ser Gln Phe Ala Ser Asn Pro Leu Val Ile Phe Asp Thr
   145 150 155 160
   Asn Asn Glu Tyr His Asp Met Asp Gln Thr Leu Val Leu Asn Leu Asn
   165 170 175
   Gln Ala Ala Ile Asp Gly Ile Arg Ser Ala Gly Ala Thr Ser Gln Tyr
   180 185 190
   Ile Phe Val Glu Gly Asn Ser Trp Thr Gly Ala Trp Thr Trp Thr Asn
   195 200 205
   Val Asn Asp Asn Met Lys Ser Leu Thr Asp Pro Ser Asp Lys Ile Ile
   210 215 220
   Tyr Glu Met His Gln Tyr Leu Asp Ser Asp Gly Ser Gly Thr Ser Ala
   225 230 235 240
   Thr Cys Val Ser Ser Thr Ile Gly Gln Glu Arg Ile Thr Ser Ala Thr
   245 250 255
   Gln Trp Leu Arg Ala Asn Gly Lys Lys Gly Ile Ile Gly Glu Phe Ala
   260 265 270
   Gly Gly Ala Asn Asp Val Cys Glu Thr Ala Ile Thr Gly Met Leu Asp
   275 280 285
   Tyr Met Ala Gln Asn Thr Asp Val Trp Thr Gly Ala Ile Trp Trp Ala
   290 295 300
   Ala Gly Pro Trp Trp Gly Asp Tyr Ile Phe Ser Met Glu Pro Asp Asn
   305 310 315 320
   Gly Ile Ala Tyr Gln Gln Ile Leu Pro Ile Leu Thr Pro Tyr Val Pro
   325 330 335
   Gly Leu Asp Gly Ser Asn Pro Gly Asn Pro Thr Thr Thr Val Val Pro
   340 345 350
   Pro Ala Ser Thr Ser Thr Ser Arg Pro Thr Ser Ser Thr Ser Ser Pro
   355 360 365
   Val Ser Thr Pro Thr Gly Gln Pro Gly Gly Cys Thr Thr Gln Lys Trp
   370 375 380
   Gly Gln Cys Gly Gly Ile Gly Tyr Thr Gly Cys Thr Asn Cys Val Ala
   385 390 395 400
   Gly Thr Thr Cys Thr Gln Leu Asn Pro Trp Tyr Ser Gln Cys Leu
   405 410 415
<210> 5
   <211> 1211
   <212> DNA
   <213> Acremonium thermophilum
<220>
   <221> gene
   <222> (13)..(1206)
<220>
   <221> Intron
   <222> (96)..(154)
<220>
   <221> Intron
   <222> (404)..(526)
<220>
   <221> Intron
   <222> (1134)..(1197)
<400> 5
   ggactgcgca tcatgcgctc ctcacccttt ctccgcgcag ctctggctgc cgctctgcct     60
   ctgagcgccc atgccctcga cggaaagtcg acgaggtatg ccaatcctcg tacctctgcc    120
   ctctgtagaa acaagtgacc gactgcaaag acagatactg ggactgctgc aagccgtcct    180
   gcggctgggc cggaaaggcc tcggtgaacc agcccgtctt ctcgtgctcg gccgactggc    240
   agcgcatcag cgacttcaac gcgaagtcgg gctgcgacgg aggctccgcc tactcgtgcg    300
   ccgaccagac gccctgggcg gtcaacgaca acttctcgta cggcttcgca gccacggcca    360
   tcgccggcgg ctccgagtcc agctggtgct gcgcctgcta tgcgtgagtt ctctgcaagc    420
   cgcttcccac ccccgctttc tgtgcaggcc gcttcccccc tacccaccca cttccccccc    480
   cccgcctctg tgatcgggca tccgagctaa gttgcgtgtc gtccagactc accttcaact    540
   cgggccccgt cgcgggcaag accatggtgg tgcagtcgac cagcaccggc ggcgacctgg    600
   gcagcaacca gttcgacctc gccatccccg gcggcggcgt gggcatcttc aacggctgcg    660
   cctcccagtt cggcggcctc cccggcgccc agtacggcgg catcagcgac cgcagccagt    720
   gctcgtcctt ccccgcgccg ctccagccgg gctgccagtg gcgcttcgac tggttccaga    780
   acgccgacaa ccccaccttc accttccagc gcgtgcagtg cccgtccgag ctcacgtccc    840
   gcacgggctg taagcgcgac gacgacgcca gctatcccgt cttcaacccg cctagcgtcc    900
   ctggccttga cggcagcaac cccggcaacc cgaccaccac cgtcgttcct cccgcttcta    960
   cctccacctc ccgtccgacc agcagcacta gctctcccgt ttcgaccccg actggccagc   1020
   ccggcggctg caccacccag aagtggggcc agtgcggcgg tatcggctac accggctgca   1080
   ctaactgcgt tgctggcacc acctgcactc agctcaaccc ctggtacagc caggtatgtt   1140
   tctcttcccc cttctagact cgcttggatt tgacagttgc taacatctgc tcaacagtgc   1200
   ctgtaactgc a                                                         1211
<210> 6
   <211> 315
   <212> PRT
   <213> Acremonium thermophilum
<400> 6
   Met Arg Ser Ser Pro Phe Leu Arg Ala Ala Leu Ala Ala Ala Leu Pro
   1               5                   10                  15
   Leu Ser Ala His Ala Leu Asp Gly Lys Ser Thr Arg Tyr Trp Asp Cys
               20                  25                  30
   Cys Lys Pro Ser Cys Gly Trp Ala Gly Lys Ala Ser Val Asn Gln Pro
           35                  40                  45
   Val Phe Ser Cys Ser Ala Asp Trp Gln Arg Ile Ser Asp Phe Asn Ala
       50                  55                  60
   Lys Ser Gly Cys Asp Gly Gly Ser Ala Tyr Ser Cys Ala Asp Gln Thr
   65                  70                  75                  80
   Pro Trp Ala Val Asn Asp Asn Phe Ser Tyr Gly Phe Ala Ala Thr Ala
                   85                  90                  95
   Ile Ala Gly Gly Ser Glu Ser Ser Trp Cys Cys Ala Cys Tyr Ala Leu
               100                 105                 110
   Thr Phe Asn Ser Gly Pro Val Ala Gly Lys Thr Met Val Val Gln Ser
           115                 120                 125
   Thr Ser Thr Gly Gly Asp Leu Gly Ser Asn Gln Phe Asp Leu Ala Ile
       130                 135                 140
   Pro Gly Gly Gly Val Gly Ile Phe Asn Gly Cys Ala Ser Gln Phe Gly
   145                 150                 155                 160
   Gly Leu Pro Gly Ala Gln Tyr Gly Gly Ile Ser Asp Arg Ser Gln Cys
                   165                 170                 175
   Ser Ser Phe Pro Ala Pro Leu Gln Pro Gly Cys Gln Trp Arg Phe Asp
               180                 185                 190
   Trp Phe Gln Asn Ala Asp Asn Pro Thr Phe Thr Phe Gln Arg Val Gln
           195                 200                 205
   Cys Pro Ser Glu Leu Thr Ser Arg Thr Gly Cys Lys Arg Asp Asp Asp
       210                 215                 220
   Ala Ser Tyr Pro Val Phe Asn Pro Pro Ser Val Pro Gly Leu Asp Gly
   225                 230                 235                 240
   Ser Asn Pro Gly Asn Pro Thr Thr Thr Val Val Pro Pro Ala Ser Thr
                   245                 250                 255
   Ser Thr Ser Arg Pro Thr Ser Ser Thr Ser Ser Pro Val Ser Thr Pro
               260                 265                 270
   Thr Gly Gln Pro Gly Gly Cys Thr Thr Gln Lys Trp Gly Gln Cys Gly
           275                 280                 285
   Gly Ile Gly Tyr Thr Gly Cys Thr Asn Cys Val Ala Gly Thr Thr Cys
       290                 295                 300
   Thr Gln Leu Asn Pro Trp Tyr Ser Gln Cys Leu
   305                 310                 315
<210> 7
   <211> 1663
   <212> DNA
   <213> Chaetomium thermophilum
<220>
   <221> gene
   <222> (1)..(1663)
<220>
   <221> Intron
   <222> (1591)..(1654)
<400> 7
   atgatgtata agaagttcgc cgctctcgcc gccctcgtgg ctggcgcctc cgcccagcag      60
   gcttgctccc tcaccgctga gaaccaccct agcctcacct ggaagcgctg cacctctggc     120
   ggcagctgct cgaccgtgaa cggcgccgtc accatcgatg ccaactggcg ctggactcac     180
   accgtctccg gctcgaccaa ctgctacacc ggcaaccagt gggatacctc cctctgcact     240
   gatggcaaga gctgcgccca gacctgctgc gtcgatggcg ctgactactc ttcgacctat     300
   ggtatcacca ccagcggtga ctccctgaac ctcaagttcg tcaccaagca ccagtacggc     360
   accaacgtcg gctcccgtgt ctatctgatg gagaacgaca ccaagtacca gatgttcgag     420
   ctcctcggca acgagttcac cttcgatgtc gatgtctcca acctgggctg cggtctcaac     480
   ggcgccctct acttcgtttc catggatgct gatggtggca tgagcaaata ctctggcaac     540
   aaggctggcg ccaagtacgg taccggctac tgcgatgctc agtgcccgcg cgacctcaag     600
   ttcatcaacg gcgaggccaa cgttgggaac tggaccccct cgaccaacga tgccaacgcc     660
   ggcttcggcc gctatggcag ctgctgctct gagatggatg tctgggaggc caacaacatg     720
   gctactgcct tcactcctca cccttgcacc accgttggcc agagccgctg cgaggccgac     780
   acctgcggtg gcacctacag ctctgaccgc tatgctggtg tttgcgaccc tgatggctgc     840
   gacttcaacg cctaccgcca aggcgacaag accttctacg gcaagggcat gactgtcgac     900
   accaacaaga agatgaccgt cgtcacccag ttccacaaga actcggctgg cgtcctcagc     960
   gagatcaagc gcttctacgt ccaggacggc aagatcattg ccaacgctga gtccaagatc    1020
   cccggcaacc ccggaaactc cattacccag gagtattgcg atgcccagaa ggtcgccttc    1080
   agtaacaccg atgacttcaa ccgcaagggc ggtatggctc agatgagcaa ggccctcgca    1140
   ggccccatgg tcctggtcat gtccgtctgg gatgaccact acgccaacat gctctggctc    1200
   gactcgacct accccatcga ccaggccggc gcccccggcg ccgagcgcgg tgcttgcccg    1260
   accacctccg gtgtccctgc cgagatcgag gcccaggtcc ccaacagcaa cgtcatcttc    1320
   tccaacatcc gtttcggccc catcggctcg accgtccctg gccttgacgg cagcaacccc    1380
   ggcaacccga ccaccaccgt cgttcctccc gcttctacct ccacctcccg tccgaccagc    1440
   agcactagct ctcccgtttc gaccccgact ggccagcccg gcggctgcac cacccagaag    1500
   tggggccagt gcggcggtat cggctacacc ggctgcacta actgcgttgc tggcaccacc    1560
   tgcactcagc tcaacccctg gtacagccag gtatgtttct cttccccctt ctagactcgc    1620
   ttggatttga cagttgctaa catctgctca acagtgcctg taa                      1663
<210> 8
   <211> 1076
   <212> DNA
   <213> Acremonium thermophilum
<220>
   <221> gene
   <222> (1)..(1076)
<220>
   <221> Intron
   <222> (84)..(142)
<220>
   <221> Intron
   <222> (392)..(514)
<400> 8
   atgcgctcct caccctttct ccgcgcagct ctggctgccg ctctgcctct gagcgcccat 60
   gccctcgacg gaaagtcgac gaggtatgcc aatcctcgta cctctgccct ctgtagaaac 120
   aagtgaccga ctgcaaagac agatactggg actgctgcaa gccgtcctgc ggctggccgg 180
   gaaaggcctc ggtgaaccag cccgtcttct cgtgctcggc cgactggcag cgcatcagcg 240
   acttcaacgc gaagtcgggc tgcgacggag gctccgccta ctcgtgcgcc gaccagacgc 300
   cctgggcggt caacgacaac ttctcgtacg gcttcgcagc cacggccatc gccggcggct 360
   ccgagtccag ctggtgctgc gcctgctatg cgtgagttct ctgcaagccg cttcccaccc 420
   ccgctttctg tgcaggccgc ttccccccta cccacccact tccccccccc cgcctctgtg 480
   atcgggcatc cgagctaagt tgcgtgtcgt ccagactcac cttcaactcg ggccccgtcg 540
   cgggcaagac catggtggtg cagtcgacca gcaccggcgg cgacctgggc agcaaccagt 600
   tcgacctcgc catccccggc ggcggcgtgg gcatcttcaa cggctgcgcc tcccagttcg 660
   gcggcctccc cggcgcccag tacggcggca tcagcgaccg cagccagtgc tcgtccttcc 720
   ccgcgccgct ccagccgggc tgccagtggc gcttcgactg gttccagaac gccgacaacc 780
   ccaccttcac cttccagcgc gtgcagtgcc cgtccgagct cacgtcccgc acgggctgta 840
   agcgcgacga cgacgccagc tatcccgtct tcaacccgcc tagcggtggc tcccccagca 900
   ccaccagcac caccaccagc tccccgtccg gtcccacggg caaccctcct ggaggcggtg 960
   gctgcactgc ccagaagtgg gcccagtgcg gcggcactgg cttcacgggc tgcaccacct 1020
   gcgtctcggg caccacctgc caggtgcaga accagtggta ttcccagtgt ctgtga 1076
<210> 9
   <211> 45
   <212> DNA
   <213> Thermoascus aurantiacus
<400> 9
   attaaccgcg gactgcgcat catgaagctc ggctctctcg tgctc 45
<210> 10
   <211> 29
   <212> DNA
   <213> Thermoascus aurantiacus
<400> 10
   aactgaggca tagaaactga cgtcatatt 29
<210> 11
   <211> 28
   <212> DNA
   <213> Chaetomium thermophilum
<400> 11
   taatttacgt acctggcctt gacggcag 28
<210> 12
   <211> 30
   <212> DNA
   <213> Chaetomium thermophilum
<400> 12
   attaactgca gttacaggca ctgttgagca 30
<210> 13
   <211> 42
   <212> DNA
   <213> Acremonium thermophilum
<400> 13
   attaaccgcg gactgcgcat catgcgctcc tcaccctttc tc 42
<210> 14
   <211> 33
   <212> DNA
   <213> Acremonium thermophilum
<400> 14
   ttaatctgca gtcacagaca ctgggaatac cac 33
<210> 15
   <211> 81
   <212> PRT
   <213> Chaetomium thermophilum
<400> 15
   Val Pro Gly Leu Asp Gly Ser Asn Pro Gly Asn Pro Thr Thr Thr Val
   1               5                   10                  15
   Val Pro Pro Ala Ser Thr Ser Thr Ser Arg Pro Thr Ser Ser Thr Ser
               20                  25                  30
   Ser Pro Val Ser Thr Pro Thr Gly Gln Pro Gly Gly Cys Thr Thr Gln
           35                  40                  45
   Lys Trp Gly Gln Cys Gly Gly Ile Gly Tyr Thr Gly Cys Thr Asn Cys
       50                  55                  60
   Val Ala Gly Thr Thr Cys Thr Gln Leu Asn Pro Trp Tyr Ser Gln Cys
   65                  70                  75                  80
   Leu